# EUROPEAN PATENT SPECIFICATION

(11) **EP 4 061 339 B1**
(45) Date of publication and mention of the grant of the patent: **17.06.2026**
(21) Application number: 20815906.1
(22) Date of filing: 19.11.2020
(51) Int. Cl.: A61K 9/107, A61K 47/44, A61K 31/352, A61P 29/00

(54) **GELLED OIL-IN-WATER EMULSION COMPRISING AT LEAST ONE CANNABINOID**
GELIERTE ÖL-IN-WASSER-EMULSION MIT MINDESTENS EINEM CANNABINOID
ÉMULSION D'HUILE DANS EAU GÉLIFIÉE COMPRENANT AU MOINS UN CANNABINOÏDE

(30) Priority: 21.11.2019 GB 201916960
(43) Date of publication of application: 28.09.2022
(73) Proprietor: Vitux Group AS, 0667 Oslo (NO)
(72) Inventor: LUNDHAUG, Kamilla, 0667 Oslo (NO); SIWEK, Andrzej, 0667 Oslo (NO); DRAGET, Kurt, 0667 Oslo (NO)
(74) Representative: Dehns
(86) International application number: PCT/GB2020/052958
(87) International publication number: WO 2021/099792

(56) References cited:
- EP-A1- 3 178 470
- WO-A1-2019/135224
- WO-A1-2019/135225
- WO-A2-2010/041015

## Description

### Field of the invention

The present invention relates to compositions for oral administration which contain at least one cannabinoid, to methods for their preparation and to their use as pharmaceuticals or nutraceuticals. More specifically, it relates to such compositions in the form of gelled oil-in-water emulsions.

### Background of the invention

Cannabinoids are chemical compounds obtained from cannabis that interact with receptors in the brain and body to produce various effects. Cannabis is a genus of plants in the Cannabaceae family and includes *Cannabis sativa, Cannabis indica* and *Cannabis ruderalis.* All cannabis plants produce active cannabinoid compounds, a class of mono- to tetracyclic C21 or C22 meroterpenoids, but each variety produces the compounds in different concentrations and proportions which are not only dependent on genomic background, but also on growing conditions and climate. More than 100 different cannabinoids can be isolated from *Cannabis sativa,* but perhaps the most notable is the psychoactive compound delta-9-tetrahydrocannabinol (THC). Other pharmacologically important cannabinoids include cannabidiol (CBD), cannabinol (CBN), cannabinoid acids, cannabigerol, and the cannabivarins. CBD may be extracted from plant parts of *Cannabis sativa* and is often administered as a CBD-containing hemp oil which contains other cannabinoids in varying amounts. CBD can also be isolated to provide a higher value product, for example for pharmaceutical use where a purified and well-defined active ingredient is required. The need for high purity CBD can also be met by chemical synthesis of the compound.

Recently, there has been renewed interest in the therapeutic potential of cannabinoids, especially cannabidiol (CBD) and delta-9-tetrahydrocannabinol (THC), and the regulatory landscape for cannabinoids is undergoing rapid change. THC is generally regarded as a narcotic drug, but is permitted for medical use in some countries. Certain CBD formulations are already approved for use as pharmaceuticals and it is expected that more products will gain regulatory approval as further clinical research confirms the use of this particular group of compounds. Cannabinoid medicines approved for medical use include: Nabiximols (Sativex^{®}) - an oromucosal spray containing THC and CBD in ethanol for the relief of moderate to severe multiple sclerosis-caused spasticity in adults, and for use as an analgesic in the relief of cancer pain; Nabilone (Cesamet^{™}) - an oral capsule containing a synthetic cannabinoid similar to THC for use in the control of nausea and vomiting associated with cancer chemotherapy; and Dronabinol (Marinol^{®}) - oral capsules or an oral solution containing synthetic THC for appetite stimulation and for the treatment of chemotherapy-induced nausea in patients with AIDS. Epidiolex, a liquid formulation of a CBD solution, has also recently been approved in the US for the treatment of seizures associated with two rare and severe forms of epilepsy.

Cannabinoid-containing extracts may be obtained from cannabis using a range of extraction methods (e.g. using supercritical CO₂, hydrocarbons, alcohols, etc.), and may be further purified, for example by precipitation in alcohols, by distillation or by chromatographic separation. The resulting cannabinoids are highly lipophilic and practically insoluble in water. This makes them difficult to deliver using many traditional pharmaceutical drug delivery methods. A common problem is administration which can provide a controllable and/or systemic bioavailability of the active cannabinoid compounds.

To date, oral delivery of the cannabinoids has generally been in the form of solid dosage formulations, or as oral solutions. Due to variable absorption and extensive first-pass metabolism (i.e. absorption by the liver), however, the bioavailability of such orally delivered cannabinoids is low. This means that significant doses are required to obtain the desired effects. Oral delivery of cannabinoids in lipid formulations has been proposed and is generally considered to increase absorption. However, cannabinoids are very slowly absorbed from the gastrointestinal tract with maximum plasma concentrations achieved from 1 to 6 hours after administration. Delivery to the gastrointestinal tract also does not address the first-pass metabolism effect which reduces blood levels. Sublingual delivery, for example in the form of sublingual sprays or aerosols, has also been suggested. Although this provides faster absorption and a reduced first-pass effect when compared to other oral delivery routes, it can be difficult to control the delivered dose and this method of delivery is not convenient for all patients, particularly children and the elderly.

Alternative delivery routes have been investigated. These include delivery to the lungs by smoking cannabinoid containing plant parts, or by vaporizing solid or liquid extracts (which may be partially or highly purified) by the application of heat. Delivery via the lungs is highly effective in rapid delivery of the actives systemically into the blood, but is not without its problems. One problem with heating cannabinoids is that this can change their composition (e.g. from acid to non-acid form). It may, for example, change THC acid to the non-acid form, tetrahydrocannabinol, which is substantially more psychotropic. In the case of CBD, which is not psychotropic, the structurally different acid version of the material, cannabidiolic acid, has shown increased *in vivo* activity compared to CBD itself. These methods also have poor control over the dose of active material. When delivered by smoking, for example, patients who are heavy, experienced smokers show a more than two-fold higher systemic bioavailability of the cannabinoids than light, occasional smokers due to a higher efficiency of their inhalation technique. The use of smoking and vaporizing as delivery methods are thus generally considered disadvantageous.

The use of metered dose inhalers (MDIs) in which a pressurized aerosol composition containing a cannabinoid is atomized and delivered direct to the lungs has also been proposed, but this can result in irritation of the upper airways and often leads to coughing which can influence the efficiency of the inhalation process and the resulting bioavailability. The delivery of an accurate dose of the cannabinoid is also difficult to control since it is dependent on the depth of breath and length of inhalation.

As highly lipophilic molecules, cannabinoids are susceptible to degradation, especially when presented in solution, via the action of light and temperature as well as auto-oxidation. Formulation thus plays a crucial role in increasing bioavailability and physicochemical stability of the compounds. Packaging of the formulation containing the active compounds also impacts physicochemical stability of the product. Strategies which have been adopted to address these problems include salt formation (pH adjustment), the use of co-solvents (e.g. ethanol), nano- and micro-emulsification, and encapsulation in lipid-based formulations (e.g. liposomes) and in nanoparticles. The delivery of cannabinoids in the form of lipid nanoparticles has been suggested, but when used in solution these have a tendency to re-aggregate, i.e. to coalesce.

Thus there is a need for other delivery forms for cannabinoids, in particular delivery forms having at least one of the following attributes: the ability to deliver a defined dose of the active cannabinoid components; the ability to provide rapid delivery of the active cannabinoids into the blood stream thus substantially avoiding the first-pass metabolism effect; and ease of administration in order to ensure a high level of patient compliance, especially in the elderly and in pediatric patients. The latter is important since many of the conditions presently under investigation for treatment using cannabinoids are most commonly encountered in children (e.g. Dravet syndrome, ADHD, autism, etc.) or in the elderly population (e.g. Alzheimer's disease, dementia, and Parkinson's disease).

WO 2019/135224 A1 discloses taste-enhanced cannabinoid submicron emulsion syrup compositions. The compositions comprise at least one cannabinoid in a pharmaceutically acceptable carrier, at least one triglyceride oil, at least two emulsifiers, at least one taste-enhancing excipient and water. WO 2019/135225 A1 discloses solid self-emulsifying cannabinoid compositions comprising at least one cannabinoid, at least one essential oil or at least one terpene or a mixture thereof, at least two emulsifiers, and at least one adsorbing powder. WO 2010/041015 A2 discloses an oral pharmaceutical composition in unit dose form comprising a lipophilic drug substance within a unitary carrier body, said body comprising a soft, chewable, gelled oil-in-water emulsion. EP 3178470 A1 discloses an oral pharmaceutical composition, optionally in unit dose form, comprising a lipophilic drug substance in dispersion in a physiologically tolerable aqueous carrier, preferably an aqueous gel.

### Summary of the invention

The Applicant now proposes that cannabinoids can be orally administered in the form of a "gelled" oil-in-water emulsion as herein described. Specifically, they propose the delivery of lipophilic cannabinoids as a component of the oil phase of such an emulsion.

The provision of active cannabinoids in an oil-in-water emulsion which is "gelled" as herein described provides a stable emulsion which effectively prevents (or at least minimises) coalescence of the oil droplets. It also serves to protect the cannabinoids from light and air (i.e. oxygen), thereby reducing their potential oxidation.

In at least certain embodiments, the use of a "gelled" oil-in-water emulsion as herein described also enables the provision of a formulation containing micro- and/or nanoparticles of oil carrying the highly lipophilic cannabinoid material. Oil droplets in this size range, particularly those on the nanoscale, can significantly enhance oromucosal delivery (whether sublingual or buccal) of the active cannabinoids thereby reducing the first-pass metabolism effect.

The good organoleptic characteristics (i.e. pleasant taste and mouthfeel) of the "gelled" oil-in-water emulsions herein described has the advantage of encouraging retention of the product in the mouth which further aids in oromucosal delivery of the active cannabinoid material.

According to the invention provides an orally administrable, gelled oil-in-water emulsion in unit dose form, wherein the emulsion is a self-supporting, viscoelastic solid having a gelled aqueous phase and an oil phase which comprises one or more physiologically tolerable lipids and at least one cannabinoid, and wherein the oil phase constitutes from 20 to 50 wt.% of the emulsion.

In another aspect the invention provides a method for the preparation of a gelled oil-in-water emulsion in unit dose form as herein defined, said method comprising the steps of: forming an oil phase which comprises one or more physiologically tolerable lipids and at least one cannabinoid; forming an aqueous phase comprising a physiologically tolerable gelling agent; combining said oil phase and said aqueous phase to form an oil-in-water emulsion; and allowing said emulsion to gel.

In another aspect the invention provides a method for the preparation of a gelled oil-in-water emulsion in unit dose form as herein defined, said method comprising the steps of: forming an oil phase which comprises one or more physiologically tolerable lipids; forming an aqueous phase comprising a physiologically tolerable gelling agent; combining said oil phase, said aqueous phase and at least one cannabinoid to form an oil-in-water emulsion; and allowing said emulsion to gel.

In a further aspect the invention provides a gelled oil-in-water emulsion as herein defined for oral use as a medicament or for oral use in therapy.

In another aspect the invention provides a gelled oil-in-water emulsion as herein defined for oral use in the treatment of a condition responsive to a cannabinoid, wherein said condition is selected from pain (e.g. cancer pain, arthritis pain), inflammation, neurological disorders, mood disorders (e.g. anxiety), epilepsy, sleep disorders, the symptoms of multiple sclerosis, anorexia, schizophrenia, inflammatory bowel disease (e.g. Crohn's disease and ulcerative colitis), and chemotherapy-induced nausea.

In another aspect the invention provides the non-therapeutic use of a gelled oil-in-water emulsion as herein defined as a nutraceutical.

### Detailed description of the invention

### Definitions

The term "gel" refers to a form of matter that is intermediate between a solid and a liquid. The formation of a "gel" will typically involve the association or cross-linking of polymer chains to form a three-dimensional network that traps or immobilises solvent (e.g. water) within it to form a sufficiently rigid structure that is resistant to flow at ambient temperature, i.e. at a temperature below about 25°C, preferably below about 20°C. In rheological terms, a "gel" may be defined according to its storage modulus (or "elastic modulus"), G', which represents the elastic nature (energy storage) of a material, and its loss modulus (or "viscous modulus"), G", which represents the viscous nature (energy loss) of a material. Their ratio, tan δ (equal to G"/G'), also referred to as the "loss tangent", provides a measure of how much the stress and strain are out of phase with one another.

A material which is "viscoelastic" is characterised by rheological properties which resemble, in part, the rheological behaviour of a viscous fluid and, also in part, that of an elastic solid.

The gelled oil-in-water emulsions according to the invention are "self-supporting, viscoelastic solids". This is intended to mean that they exhibit characteristics intermediate between those of a solid and a liquid, but have a dominant solid behaviour, i.e. they have rheological characteristics more similar to that of a solid than a liquid. A "solid dominant behaviour" cannot be diluted away (i.e. destroyed) by adding more solvent. In contrast, in the case of a weak (or entangled) gel lacking stable (i.e. long lived) intermolecular crosslinks, the entangled network structure of the gel can be removed by adding more solvent and can readily be destroyed even at very low shear rate/shear stress.

The gelled oil-in-water emulsions of the invention exhibit mechanical rigidity, yet in contrast to a solid they are deformable. Specifically, the gelled emulsions herein described have a storage modulus, G', which is greater than their loss modulus, G", (i.e. G' > G") over a wide frequency range, for example in the frequency range from 0.001 to 10 Hz when measured at ambient temperature (i.e. at a temperature in the range of 18°C to 25°C, e.g. at 20°C) and 0.1% strain.

Storage modulus and loss modulus may be measured using known methods, for example using a Kinexus Ultra+ Rheometer applying a C 4/40 measuring geometry. Storage modulus and loss modulus values are not expected to differ when measured using other types of rheometer within the linear viscoelastic range.

More specifically, the gelled oil-in-water emulsions herein described will have the following properties: G' > G" over a frequency range of 0.001 to 10 Hz at 0.1% strain; and a storage modulus (G') at ambient temperature (i.e. at a temperature in the range of 18°C to 25°C, e.g. at 20°C) in the range from 10 to 200,000 Pa, preferably 100 to 100,000 Pa, more preferably 500 to 50,000 Pa.

Weak gels will typically have a loss tangent, tan δ > 0.1. For strong gels, or fully developed gels, G' >> G" and lower tan δ values (< 0.1) are observed. The gelled oil-in-water emulsions herein described would generally be considered "strong gels" at ambient temperature, i.e. at a temperature in the range of 18°C to 25°C, e.g. at 20°C.

As used herein, the term "gelled" refers to the formation of a "gel". The term is used herein both in relation to the physical nature of the aqueous phase of the emulsion and that of the oil-in-water emulsion. As will be understood, the oil droplets act more or less like a solid when dispersed throughout the gelled aqueous phase of the oil-in-water emulsions which are the subject of the invention. The "gelled" nature of the aqueous phase is thus also a characteristic of the oil-in-water emulsion, i.e. it can also be considered "gelled" as described herein.

Unless otherwise defined, the term "liquid" as used herein refers to a substance which flows freely and which maintains a constant volume. It includes thickened liquids and viscous liquids which flow. A "liquid" has a loss modulus (G") which is greater than its storage modulus (G') and a loss tangent (tan δ) which is greater than 1.

As used herein, the term "cannabinoid" refers to a chemical compound that acts on one or more cannabinoid receptors which are part of the endocannabinoid system found in cells that alter neurotransmitter release in the brain. The cannabinoid receptors include type 1 and type 2, termed CB₁ and CB₂. The class of cannabinoids includes all major and minor cannabinoids found in natural cannabis and hemp material that can be isolated or which can be produced synthetically, i.e. manufactured. It also includes any natural or synthetic derivatives (e.g. acid derivatives) of such compounds, including metabolites. Any pharmaceutically acceptable salts of such compounds are also included.

As used herein, the term "fatty acid" refers to an un-branched or branched, preferably un-branched, hydrocarbon chain having a carboxylic acid (-COOH) group at one end, conventionally denoted the α (alpha) end. The hydrocarbon chain may be saturated or (mono- or poly-) unsaturated. By convention, the numbering of the carbon atoms starts from the α-end such that the carbon atom of the carboxylic acid group is carbon atom number 1. The other end, which is usually a methyl (-CH₃) group, is conventionally denoted ω (omega) such that the terminal carbon atom is the ω-carbon. Any double bonds present may be cis- or trans- in configuration. The nomenclature "ω-x" is used to signify that a double bond is located on the xth carbon-carbon bond, counting from the terminal carbon (i.e. the ω-carbon) towards the carbonyl carbon.

By "physiologically tolerable" is meant any component which is suitable for administration to a human or non-human animal body, in particular which is suitable for oral administration.

By "pharmaceutical" is meant any product intended for a medical purpose, e.g. for treating or preventing any disease, condition or disorder of a human or non-human animal body, or for preventing its recurrence, or for reducing or eliminating the symptoms of any such disease, condition or disorder. The use and production of a product as a "pharmaceutical" will be closely regulated by a government agency. It may, but need not, be prescribed by a physician. For example, it may be available "over the counter", i.e. without a prescription.

"Treatment" or "treating" includes any therapeutic application that can benefit a human or non-human animal (e.g. a non-human mammal). Both human and veterinary treatments are within the scope of the present invention, although primarily the invention is aimed at the treatment of humans. Veterinary treatment includes the treatment of livestock and domestic animals (e.g. pets such as cats, dogs, rabbits, etc.). Treatment may be in respect of an existing disorder or it may be prophylactic.

In contrast to a pharmaceutical, a "nutraceutical" need not be the subject of regulatory approval. The term "nutraceutical" is used herein to refer to a product which is generally considered beneficial to maintain or augment the health and/or general well-being of a human or non-human animal subject. Such substances include, in particular, dietary supplements such as vitamins and minerals which are intended to augment the health of a subject (e.g. a human subject).

As will be understood, some substances may be considered both a "pharmaceutical" and a "nutraceutical". Categorization of a substance as one or the other, or indeed both, may vary in different countries depending on local regulations relating to medicinal products. It may also be dependent on the recommended daily dosage of any given substance. Higher daily doses of certain vitamins such as vitamin D, for example, may be regulated as a pharmaceutical whereas lower daily dosages may be considered nutraceutical.

By "a pharmaceutical composition" is meant a composition in any form suitable to be used for a pharmaceutical purpose.

By a "nutraceutical composition" is meant a composition in any form suitable to be used for a nutraceutical purpose.

A "pharmaceutically effective amount" relates to an amount that will lead to the desired pharmacological and/or therapeutic effect, i.e. an amount of the agent which is effective to achieve its intended pharmaceutical purpose. While individual patient needs may vary, determination of optimal ranges for effective amounts of any active agent is within the capability of those skilled in the art.

A "nutraceutically effective amount" relates to an amount that will lead to the desired nutraceutical effect, i.e. an amount of the agent which is effective to achieve its intended nutraceutical purpose. While the individual needs of a subject may vary, determination of optimal ranges for effective amounts of any active agent is within the capability of those skilled in the art.

The term "capsule" is used herein to refer to a unitary dosage form having a casing or coating (herein referred to as the "capsule shell") which encloses a gelled oil-in-water emulsion as herein defined.

As used herein, "water activity" is the partial vapour pressure of water in a composition at a specified temperature divided by the standard state partial vapour pressure of water at the same temperature. Water activity thus acts as a measure of the amount of free (i.e. unbound) water in a composition. Water activity may be measured by methods known to those skilled in the art, for example by using a Rotronic Hygrolab instrument.

In a first aspect the invention provides an orally administrable, gelled oil-in-water emulsion in unit dose form, wherein the emulsion is a self-supporting, viscoelastic solid having a gelled aqueous phase and an oil phase which comprises one or more physiologically tolerable lipids and at least one cannabinoid, and wherein the oil phase constitutes from 20 to 50 wt.% of the emulsion.

The oil phase of the emulsion will comprise a physiologically tolerable lipid, or a mixture of different physiologically tolerable lipids, which acts as a carrier for the lipophilic cannabinoid material. The cannabinoid material will generally be dissolved in the oil phase.

Cannabinoids which may be used in the invention include any known cannabinoid, or combination of known cannabinoids, found in Cannabis sp., especially in *Cannabis sativa,* or any cannabinoid which is synthetically produced. Derivatives of any such cannabinoids may also be used in the invention including, but not limited to, acid derivatives.

The cannabinoids for use in the invention may be extracted from cannabis, or they may be synthetically produced. Extraction methods are well known in the art and include, for example, the use of supercritical CO₂, hydrocarbons, and alcohols. Following extraction, a mixture of active cannabinoids will typically be produced. Depending on the extraction method used (e.g. the nature of the solvent used for extraction), this material may be used without further purification, i.e. as an "extract", or it may be further purified using known methods such as precipitation in alcohols, by distillation or by chromatographic separation. Purification may, for example, be carried out to provide a well-defined product for pharmaceutical use.

The cannabinoid may be selected from any of the known cannabinoids. These include, without limitation, the tetrahydrocannabinols and their isomers (including delta-9-tetrahydrocannabinol and its isomers, e.g. trans(-)-delta-9-tetrahydrocannabinol), tetrahydrocannabinolic acids and their isomers (including delta-9-tetrahydrocannabinolic acid and its isomers, e.g. trans (-)-delta-9-tetrahydrocannabinolic acid), cannabidiol, cannabidiolic acid, cannabigerol, cannabigerolic acid, cannabigerovarin, cannabigerovarinic acid, cannabichromene, cannabichromenic acid, cannabidivarin, cannabidivarinic acid, cannabivarin, cannabivarinic acid, tetrahydrocannabivarin, tetrahydrocannabivarinic acid, cannabinol, cannabinolic acid, cannabinodiol, cannabielsoin, cannabicyclol, and cannabicitran, and any isomers thereof, and any mixtures thereof.

In the case where a mixture of cannabinoids is used, the major component(s) will typically be selected from one or more of delta-9-tetrahydrocannabinol, delta-9-tetrahydrocannabinolic acid, cannabidiol, cannabidiol acid, cannabichromic acid, cannabichromene, cannabigerolic acid, cannabidivarin, cannabivarinic acid, tetrahydrocannabivarinic acid, tetrahydrocannabivarin and cannabigerol, More preferably, the major component(s) will be one or more of delta-9-tetrahydrocannabinol, delta-9-tetrahydrocannabinolic acid, cannabidiol, and cannabidiol acid. Yet more preferably, the major component may be cannabidiol or its acid variant. The major cannabinoid components may be present in an amount of from 80 to 100 wt.% (based on the total weight of all cannabinoids present), for example they may be present in an amount of from 85 to 99 wt.%, preferably 90 to 98 wt.%, e.g. 95 to 98 wt.%. The use of cannabinoid materials of high purity is beneficial when a controlled dose is required, for example in a composition intended for use as a pharmaceutical.

In one embodiment, the active cannabinoid material for use in the invention will comprise a mixture of at least two (e.g. two) cannabinoids selected from the group consisting of a tetrahydrocannabinol (e.g. a delta-9-tetrahydrocannabinol), a tetrahydrocannabinolic acid (e.g. a delta-9-tetrahydrocannabinolic acid), a cannabidiol, and a cannabidiolic acid. Minor amounts of other cannabinoids may be present, but they will generally be present in an amount of less than 20 wt.%, preferably less than 10 wt.%, more preferably less than 5 wt.%, e.g. less than 2 wt.% (based on the total weight of all cannabinoids present).

In one set of embodiments, the cannabinoid material for use in the invention will comprise a tetrahydrocannabinol (e.g. a delta-9-tetrahydrocannabinol) and/or a cannabidiol. A mixture of these cannabinoids in substantially purified form may be used. Thus, the cannabinoid material for use in the invention may consist of a tetrahydrocannabinol (e.g. a delta-9-tetrahydrocannabinol) and/or a cannabidiol. The use of a combination of a tetrahydrocannabinol (e.g. a delta-9-tetrahydrocannabinol) and a cannabidiol forms a preferred embodiment.

Synthetic and semi-synthetic forms of any known cannabinoid may also be used in the invention. Examples of such agents include Dronabinol which is a synthetic delta-9 THC marketed under the tradename Marinol^{®}. Other synthetic cannabinoids include Nabilone - a synthetic cannabinoid similar to THC which is marketed as Cesamet^{®}; Dexanabinol - a synthetic non-psychotropic cannabinoid that blocks NMDA receptors and COX-2 cytokines and chemokines (manufactured by Solvay Pharmaceuticals); Cannabinor (formerly PRS-211,375) - a synthetic cannabinoid that specifically binds to CB₂ (manufactured by Pharmos); HU308 - a synthetic cannabinoid that specifically binds to CB₂ (manufactured by Pharmos); HU331 - a synthetic cannabinoid manufactured by Cayman Chemical; and CT-3 (ajulemic acid) - a more potent analogue of the THC metabolite THC-11-oic acid.

The amount of active cannabinoid material present in the compositions according to the invention will vary, for example it will be dependent on the type of cannabinoid(s), the intended use of the composition (whether pharmaceutical or nutraceutical), the intended recipient, etc. It may be varied according to need and suitable amounts may be readily determined by those skilled in the art. Where the composition is provided in unit dose form, the amount of cannabinoid(s) per unit dose may be selected based on the required single daily dose for treatment of a particular condition or for use as a nutraceutical. It may, for example, be in the range from 5 to 350 mg, preferably from 10 to 300 mg, e.g. 20 to 200 mg. For nutraceutical use, the amount of cannabinoid(s) will generally be at the lower end of these ranges, for example from 5 to 50 mg, preferably from 7 to 20 mg, more preferably from 10 to 15 mg, per unit dose.

A range of different lipids are known for oral use in pharmaceutical and/or nutraceutical products and any of these may be used in the oil phase of the emulsions herein described. Sources of lipids include plant oils, such as but not limited to, rapeseed oil, sunflower oil, corn oil, olive oil, sesame oil, palm kernel oil, coconut oil, nut oils (e.g. almond oil or peanut oil), and hemp oil.

Lipids derived from natural products typically comprise a mixture of different lipid components. In one embodiment, the oil phase will thus comprise a mixture of different lipids. For example, it may comprise a mixture of lipids having different chain lengths and/or different degrees of saturation.

Lipids for use in the invention may be liquid, solid or semi-solid at ambient temperature (i.e. at temperatures of about 18°C to about 25°C). Those which are liquid at such temperatures are generally preferred. Any combination of liquid, solid and semi-solid lipids may also be used. Solid lipids having a melting point below about 100°C, preferably below about 70°C, e.g. below about 50°C may be used in the invention. Solid lipids which may be used include butter, cocoa fat, etc. If desired, the overall melting point of the lipids which make up the oil phase may be modified by mixing different lipids, for example by mixing a solid lipid (e.g. butter) with a liquid oil. An overall melting point in the range from 45 to 50°C may be desirable.

Lipids for use in the invention include, in particular, fatty acids and their derivatives. These include both naturally occurring fatty acids and their derivatives, as well as synthetic analogues. In one embodiment, the oil phase may comprise a mixture of different fatty acids, or fatty acid derivatives.

The hydrocarbon chain of the fatty acid or fatty acid derivative may be saturated or unsaturated, and it may be un-branched or branched. Preferably, it will be un-branched. Typically the hydrocarbon chain will comprise from 4 to 28 carbon atoms, and generally it will have an even number of carbons. Fatty acids differ in their chain length and may be categorized as "short", "medium", "long", or "very long" chain fatty acids. Those having a hydrocarbon chain of 5 or fewer carbons are referred to as "short-chain fatty acids"; those with a hydrocarbon chain of 6 to 12 carbon atoms are referred to as "medium-chain fatty acids"; those with a hydrocarbon chain of 13 to 21 carbons are referred to as "long-chain fatty acids"; and those with a hydrocarbon chain of 22 carbons or more are referred to as "very long-chain fatty acids". Any of these may be used in the invention.

In one embodiment, the oil phase will comprise a saturated fatty acid, or a derivative of a saturated fatty acid including, but not limited to, any of the derivatives herein described. Medium-chain saturated fatty acids and their derivatives find particular use in the invention. Those having from 8 to 12, e.g. 8, 10 or 12, carbon atoms in the hydrocarbon chain are particularly preferred - i.e. caprylic acid (C8), capric acid (C10) or lauric acid (C12), and any derivatives thereof.

Saturated fatty acids and their derivatives for use in the invention may be naturally occurring or they may be synthetically produced. Most typically, they will be naturally occurring and thus may be used in the form of mixtures of different fatty acids and/or different fatty acid derivatives. Sources of saturated fatty acids and their derivatives include, for example, coconut oil and palm kernel oil.

In another embodiment, the oil phase may comprise an unsaturated fatty acid or derivative thereof in which the carbon chain contains one or more carbon-carbon double bonds. The double bonds may be in the cis- or trans-configuration, or any combination thereof where more than one double bond is present. Those in which the double bonds are present in the trans-configuration are generally less preferred due to the need to reduce the consumption of so-called "trans-fats" as part of a healthy diet. Fatty acids and their derivatives having cis-configuration double bonds are thus preferred. Mono- and poly-unsaturated fatty acids and their derivatives are well known in the art. Such fatty acids typically will contain 12 to 26 carbons, more typically 16 to 22 carbons, and will have a mono- or poly-unsaturated hydrocarbon chain. They include, in particular, the polyunsaturated fatty acids (PUFAs) such as the essential fatty acids.

Particularly important essential fatty acids include the ω-3, ω-6 and ω-9 fatty acids. Examples of ω-3 fatty acids include alpha-linolenic acid (ALA), stearidonic acid (SDA), eicosatrienoic acid (ETE), eicosatetraenoic acid (ETA), eicosapentaenoic acid (EPA), docosapentaenoic acid (DPA), docosahexaenoic acid (DHA), tetracosapentaenoic acid and tetracosahexaenoic acid. Examples of ω-6 fatty acids include linoleic acid, gamma-linolenic acid, eicosadienoic acid, dihomo-gamma-linolenic acid (DGLA), arachidonic acid (AA), docosadienoic acid, adrenic acid, docosapentaenoic acid, and calendic acid. Examples of ω-9 fatty acids include oleic acid, eicosenoic acid, mead acid, erucic acid and nervonic acid.

Sources of unsaturated fatty acids and their derivatives include oils obtained from various animal, fish, plant, algae, and microorganism sources. Particularly important sources are fish oils, algae oils and plant oils which are rich in ω-3, ω-6 and ω-9 fatty acids. Fish oils may, for example, be obtained from anchovies, sardines and mackerel.

Any known derivatives of the fatty acids may be used in the invention. These include, in particular, the carboxylic esters, carboxylic anhydrides, glycerides (i.e. mono-, di-, or triglycerides) and phospholipids. As used herein the term "derivatives" in the context of a fatty acid also encompasses any pharmaceutically acceptable salt of a fatty acid. Suitable salts are well known to those skilled in the art and include, but are not limited to, the lithium, sodium, potassium, ammonium, meglumine, and diethylamine salts.

Examples of carboxylic acid esters of fatty acids include compounds having a terminal -CO₂R group in which R is a straight-chained or branched alkyl group, typically a short chain alkyl, preferably a C₁₋₆ alkyl group, e.g. selected from methyl, ethyl, n-propyl, iso-propyl, n-butyl, sec-butyl and n-hexyl.

Where the fatty acid derivative is a carboxylic anhydride, it may include a terminal -CO₂COR group in which R is a straight-chained or branched alkyl group, typically a short chain alkyl, preferably a C₁₋₆ alkyl group, e.g. selected from methyl, ethyl, n-propyl, iso-propyl, n-butyl, sec-butyl and n-hexyl.

Glycerides are esters derived from glycerol and up to three fatty acids. The fatty acids present may be any of those herein described and thus they may be saturated or unsaturated, for example. In the case of di- and tri-glycerides the fatty acid components may be the same or different. For example, these may be of different chain lengths.

In one embodiment, the lipid carrier for use in the invention may comprise a medium chain triglyceride (MCT). MCTs are triglycerides with two or three medium-chain fatty acids which may be identical or different. Sources of MCTs include coconut oil and palm kernel oil, for example. The fatty acids present in MCTs are typically saturated medium chain fatty acids. MCTs from coconut oil, for example, comprise C₆₋₁₂ fatty acids, predominantly C₈ and C₁₀ fatty acids. A typical fatty acid composition of an MCT oil obtained from coconut oil may, for example, comprise: 0.1 wt.% caproic acid (C 6:0), 55 wt.% caprylic acid (C 8:0), 44.8 wt.% capric acid (C 10:0), and 0.1 wt.% lauric acid (C 12:0).

Phospholipids generally consist of a glycerol molecule linked to two fatty acids (the "tail" groups) and to a hydrophilic "head" group which consists of a phosphate group. The phosphate group may be modified by linkage to choline, ethanolamine or serine. In one embodiment, the oil phase may be constituted in whole or part by a phospholipid, in particular a marine (e.g. pelagic fish or shellfish, for example krill) phospholipid.

The amount of oil present in the compositions of the invention will be dependent on factors such as the nature of the oil, the nature and desired loading level of cannabinoid(s), etc. and can be varied according to need. The oil phase carrying the chosen cannabinoid agent(s) constitutes from 20 to 50 wt.% of the emulsion and may, for example, constitute from 30 to 50 wt.%., or from 40 to 50 wt.% of the gelled oil-in-water emulsion. A high loading level of oil can readily be achieved in the gelled emulsions herein described.

As will be understood, in the compositions of the invention the oil carrying the cannabinoid materials provides the discontinuous phase within a continuous aqueous phase which is gelled. The oil is thus dispersed throughout the gelled aqueous phase in the form of oil droplets (also referred to herein as oil "particles"). Gelling of the aqueous phase provides a stable emulsion which prevents coalescence of the droplets of oil, for example due to the prevention of physical collisions between droplets.

The size of the oil particles in the gelled oil-in-water emulsion is not particularly limited. For example, oil particles having a volume-based size in the range from about 10 nm to about 100 µm, preferably from about 50 nm to about 50 µm, in particular from about 100 nm to about 10µm, e.g. from about 750 nm to about 3 µm may be provided. Volume-based average particle sizes may range from about 100 nm to about 10 µm, preferably from about 500 nm to about 2 µm. "Volume-based average" as used herein refers to the volume moment mean or De Brouckere Mean Diameter (also known as the "D[4,3]" value). This reflects the size of those particles which constitute the bulk of the sample volume and is most sensitive to the presence of large particles in the size distribution.

An essentially homogenous size distribution of oil particles may be desirable. The D₉₀ value indicates the size value which 90% of the oil particles meet out of the entirety of all of the oil droplets. D₉₀ values may range from 500 nm to 50 µm, preferably 1 µm to 20 µm, in particular from 1 µm to 5 µm. Correspondingly, the D₅₀ and D₁₀ value, respectively, indicate the size value which 50% and 10% of the oil droplets meet out of the entirety of all of the oil droplets. D₅₀ values may range from 0.1 to 5 µm, in particular from 0.3 to 2 µm, e.g. from 0.5 to 1.5 µm. D₁₀ values may range from 0.1 to 1 µm, in particular from 0.2 to 0.5 µm.

Lipid droplet size and size distributions can be determined using methods and apparatus conventional in the art, for example using a Malvern Mastersizer 3000 (Worcestershire, UK) connected to a Hydro MV, wet dispersion unit (Malvern,Worcestershire, UK). Analysis of the data may be performed using the manufacturer's software (Mastersizer 3000, v1.0.1). Testing may be carried out by dissolving and diluting the gelled emulsion in a suitable solvent (1:100) at 50°C. Suitable solvents include Milli-Q water and a 10% (v/v) HCl solution (the latter may minimize flocculation during testing). The refractive index of water and corn oil is set to 1.33 (solvent) and 1.47 (dispersed phase), respectively, and the absorption index of the dispersed droplets set to 0.01. To avoid multiple scattering or low intensity of the scattered light, the dissolved emulsion is added to the dispersion unit (containing ~125 mL water), until an obscuration of approximately 10% is obtained.

The size and size distribution of the oil particles may be varied and this can be beneficial. For example, enhanced absorption of the oil particles (and thus improved bioavailability of the active cannabinoid material) in the mouth may be achieved when these have smaller particle sizes. Although not wishing to be bound by theory, it is considered that improved bioavailability is linked to the direct uptake of the smaller oil particles by cells without the need for lipolysis. Oil particles in the nano-range (i.e. nanoparticles) are preferred for use in enhancing oromucosal (i.e. sublingual or buccal) delivery. The use of certain lipid carrier materials as herein described may also enhance uptake into the circulatory system. For example, medium-chain triglycerides (MCTs) may be taken up directly without lipolysis, either via the oromucosal membranes or in the intestines.

Size reduction of the oil particles can be achieved by various different means, for example by mechanical processes or by chemical processes involving the selection of smaller lipid molecules, or indeed by a combination of these approaches. Mechanical reduction involves the use of shear forces to break down larger oil droplets into smaller nano-scale particles. Smaller particles may thus be produced by suitable adjustments to the method used to produce the emulsion, for example by varying the shear force and/or the duration of mixing of the oil and aqueous phases. The use of higher shear forces and/or longer mixing times will produce smaller particles of oil. Suitable shear may be achieved, for example, using a conventional homogenizer such as a rotor-stator mixer, e.g. an Ultra-turrax^{®} homogenizer. A problem often encountered in mechanical processes for the production of oil-in-water emulsions is the re-aggregation (i.e. coalescence) of the particles, but this is addressed in the invention by the use of a gelled aqueous phase which serves to stabilize the emulsion.

Chemical methods suitable for achieving a size reduction of the oil particles may involve the selection of a particular type of lipid (or combination of lipids) capable of forming smaller oil droplets. Certain oils, such as MCTs for example have a tendency to produce a finer dispersion of oil droplets. Chemical methods may also involve the use of a stabilizer, typically a surfactant or other emulsifier, which reduces the energy required for emulsification (by reducing interfacial tension) and which protects the droplets against re-aggregation. Where chemical methods are used, the use of mechanical energy may be more limited.

The oil droplets present in the gelled aqueous phase of the emulsions herein described will have a relatively high LaPlace pressure. Thus these will act more like a solid, rather than a liquid, in the final gelled emulsion. The physical state of the oil particles, particularly when these are present in nanoscale dimensions, will thus be essentially that of a solid. Such particles may be referred to as "solid lipid nanoparticles", for example.

The aqueous phase of the emulsion comprises water and at least one physiologically tolerable gelling agent. It may comprise a single gelling agent or it may comprise a mixture of different gelling agents.

Suitable gelling agents include hydrocolloids which are capable of forming gels. These comprise long chain polymers such as polysaccharides or proteins. Gelling types of hydrocolloids which may be used in the invention include, but are not limited to, alginates, pectins, carrageenans (kappa and iota), gelatin, gellan gum and agar.

Suitable gelling agents for use in the invention are well known in the food, pharmaceutical and nutraceutical industries and several are described, for example, in Phillips et al. (Ed.) "Handbook of hydrocolloids", Woodhead Publishing, Cambridge, UK, 2000, the contents of which are incorporated herein by reference. Gelling agents, or combinations of gelling agents, for use in the emulsions herein described may readily be selected by those skilled in the art taking into account factors such as the need for compatibility with the remaining components of the composition, the need for compatibility with oral delivery and the need for physiological tolerability.

The gelling agents may be materials capable of undergoing a sol-gel transformation (i.e. the transition from a liquid to a gel) under the influence of a change in physiochemical parameters such as temperature, pH, or the presence of metal ions (e.g. group I or group II metal ions).

Suitable gelling agents include, but are not limited to, saccharides (e.g. oligosaccharides and polysaccharides), proteins, and glycoproteins. Examples of gelling agents include, but are not limited to, gelatin or a mixture of gelatin and a polysaccharide, or gellan, or an alginate (e.g. sodium alginate), or a mixture of an alginate and glucono-delta-lactone (GDL). An alginate may be used alone to provide an alginic acid gel, or it may be used in combination with CaCO₃ to provide an ionically cross-linked gel.

Gelatins will readily melt when exposed to body temperatures for a sufficient period of time. When retained in the mouth at about 37°C, for example, gelatin-based compositions will thus melt over time. This may be advantageous to provide the desired release of the oil droplets in the mouth and thus enhance oromucosal (e.g. sublingual or buccal) uptake of the cannabinoid material. Melting of the gelatin in the mouth may be aided by light movement of the dose in the mouth, e.g. under the tongue or in the buccal cavity.

The gelatins used as gelling agents in the composition of the invention may be produced from the collagen of any mammal or any aquatic species (e.g. a fish). In one embodiment, the gelatin may be obtained from the collagen of a salt-water fish, for example a warm water fish. Collagen from avian sources may also provide gelatin for use in the invention.

Gelatins having an imino acid content of 5 to 25 wt.% are preferred, more especially those having an imino acid content of 10 to 25 wt.%. The gelatins will typically have a weight average molecular weight in the range 10 to 250 kDa, preferably 75 to 220 kDa, especially 80 to 200 kDa. Gelatins having Bloom values of 60-300, especially 90-260, are preferred.

Both type A and type B gelatins may be used. By "type A gelatin" is meant gelatin obtained from acid treated raw material, often from pig skin collagen or fish collagen. However, it may also be obtained from bovine sources. Gelatin obtained from acid treated warm water fish collagen is especially preferred. By "type B gelatin" is meant gelatin obtained from alkali treated raw material, typically obtained from collagen found in bovine hides and bones. Compositions comprising type B gelatins typically have much faster drug release profiles than corresponding compositions comprising type A gelatins of the same Bloom strength due to their more rapid dissolution. This effect is particularly noticeable at high Bloom strength, typically Bloom strengths above 200. The use of type B gelatins is thus preferred where immediate or very fast release of the cannabinoid is required following administration. The selection of a type B gelatin may also be desirable in order to minimise the re-aggregation of the oil particles during gel dissolution, i.e. following administration.

Lipid droplet size may be influenced by the choice of gelatin type. Appropriate selection of the gelatin type may therefore also take into account the desired lipid droplet size and distribution.

The gelatin will be present in the aqueous phase in an amount suitable to provide the desired degree of gelling as herein described. The amount will vary to some extent dependent on the type of gelatin but may readily be determined by those skilled in the art. Typically the gelatin may be present in the aqueous phase at a concentration of 1 to 50 wt.%, preferably 2 to 35 wt.%, particularly 5 to 25 wt.%, e.g. 7 to 20 wt.% or 8 to 15 wt.%, e.g. about 10 wt.% (i.e. based on the weight of the aqueous phase).

Where polysaccharides are used as the gelling agent, natural polysaccharides, synthetic polysaccharides or semi-synthetic polysaccharides, e.g. polysaccharides from plants, fish, terrestrial mammals, algae, bacteria and derivatives and fragmentation products thereof, may be used. Typical marine polysaccharides include carageenans, alginates, agars and chitosans. Typical plant polysaccharides include pectins. Typical microorganism polysaccharides include gellans and scleroglucans. The use of charged, e.g. electrostatically charged and/or sulphated polysaccharides is preferred. The use of marine polysaccharides, in particular carageenans and alginates, especially carageenans, is also generally preferred.

The carageenan family, which includes lambda-, iota- and kappa-carageenans, is a family of linear sulphated polysaccharides produced from red algae. The repeating disaccharide unit in kappa-carrageenan is β-D-galactose-4-sulphate and 3,6-anhydro-α-D-galactose, while that in iota-carrageenan is β-D-galactose-4-sulphate and 3,6-anhydro-α-D-galactose-2-sulphate. Both kappa-and iota-carrageenans are used in food preparations and find use as gelling agents in the invention.

The use of alginates, carrageenans and pectins involve cation-mediated gelation of negatively charged polysaccharides. The gelling of carrageenans, for example, is generally promoted by the inclusion of group I or group II ions, e.g. sodium, potassium or calcium ions. Both iota and kappa carrageenans form salt- or cold-setting reversible gels in an aqueous environment. Coil-helix transition and aggregation of helices form the gel network. Kappa-carrageenan has binding sites for specific monovalent cations, resulting in gel formation with decreasing shear and elastic moduli in the order Cs⁺ > K⁺ >> Na⁺ > Li⁺. As a rule, an increasing salt concentration enhances the elastic modulus and the setting and melting temperatures of a kappa-carrageenan gel. The use of water-soluble potassium, rubidium, or cesium compounds, particularly potassium compounds, and particularly naturally occurring compounds (e.g. salts) is preferred when kappa-carrageenan is used according to the invention, e.g. at concentrations of up to 100 mM, more especially up to 50 mM. A salt-dependent conformational transition is also found for iota-carrageenan. The molecules are also known to undergo coil-helix transition with strong helix-stabilisation in the presence of multivalent cations, like Ca²⁺. The use of water-soluble calcium, strontium, barium, iron or aluminium compounds, especially calcium compounds, and particularly naturally occurring compounds (e.g. salts) is preferred when iota- carrageenan is used according to the invention, e.g. at concentrations of up to 100 mM.

The polysaccharide gelling agents used according to the invention will typically have weight average molecular weights of 5kDa to 2MDa, preferably 10kDa to 1MDa, most preferably 100kDa to 900kDa, particularly 400 to 800kDa. They will typically be used at concentrations of 0.01 to 5 wt.%, preferably 0.1 to 2.5 wt.%, particularly 0.2 to 2 wt.% in the aqueous phase (i.e. based on the total weight of the aqueous phase of the emulsion). Where mono or multivalent cations, typically group I or group II metal ions, are included in the aqueous phase, this will typically be at concentrations in the range 2.5 to 100 mM, particularly 5 to 50mM.

Where pectin alone is used as the gelling agent in the aqueous phase, the pectin may be unalkoxylated / unesterified and gelled with Ca²⁺ ions, but is preferably an alkoxylated, e.g. methoxylated or ethoxylated, pectin when a high amount of solids is present and/or at low pH values. Such alkoxylated / esterified pectins, e.g. highly alkoxylated/highly esterified, for example highly methoxylated (HM) or highly ethoxylated (HE) pectins, are available commercially. By highly alkoxylated is meant a degree of esterification of at least 50%. Pectins will typically be used at concentrations of 0.01 to 5 wt.%, preferably 0.1 to 2.5 wt.%, particularly 0.2 to 2 wt.% in the aqueous phase (i.e. based on the total weight of the aqueous phase of the emulsion). Alternatively, pectins, for example unalkoxylated pectin, may be used in conjunction with a further non-proteinaceous gelling agent, for example a polysaccharide, e.g. an alginate, carrageenan or, preferably, agar.

Mixtures of polysaccharides and gelatin may be used as the gelling agent. In the case of such mixtures, the weight ratio of gelatin to polysaccharide in the aqueous phase will typically be 50:1 to 5:1, preferably 40:1 to 9:1, especially 20:1 to 10:1.

In one embodiment, the aqueous phase may have a gelling temperature in the range 10 to 45°C, more preferably 20 to 35°C. In one embodiment, it may have a melting temperature in the range 30 to 80°C, preferably 32 to 60°C, e.g. 35 to 50°C. Where a particular melting temperature is desirable, the type of gelling agent may be selected accordingly. Particularly suitable gelling agents to achieve the desired melting temperature include, but are not limited to, any of the gelatins herein described.

The aqueous phase of the gelled oil-in-water emulsion may constitute from 50 to 60 wt.% of the composition.

In addition to water, the gelling agent(s), and any required gelling initiator, other physiologically tolerable materials may also be present in the aqueous phase, for example, emulsifying agents, emulsion stabilizers, pH modifiers (e.g. buffering agents), viscosity modifiers (e.g. thickening agents, plasticizers), sweeteners, bulking agents (i.e. fillers), aromas, flavours, and colours. The nature and concentration of any such materials may readily be determined by those skilled in the art.

The presence of bulking agents (i.e. fillers) in the aqueous phase aids in reducing water activity and thus in reducing microbial growth. Water activity may, for example, be reduced to below about 0.8, for example in the range 0.5 to 0.8, or 0.6 to 0.75, or 0.65 to 0.75, e.g. in the range 0.7 to 0.75. The amount and type of bulking agents may readily be selected by those skilled in the art. Suitable examples include, but are not limited to, sugar alcohols such as sorbitol and xylitol and mixtures thereof. These may constitute from 45 to 70 wt.%, preferably 50 to 65 wt.%, e.g. 55 to 60 wt.%, based on the aqueous phase. In some cases, the selected bulking agent(s) may also act as sweetening agents depending on their concentration. For example, the compositions according to the invention may contain xylitol, e.g. as 0.5 to 50 wt.%, preferably 1 to 40 wt.%, e.g. 15 to 40 wt.%, in order to improve taste and mouthfeel.

Where a sweetener is included in the aqueous phase, this will typically be selected from natural sweeteners such as sucrose, fructose, glucose, reduced glucose, maltose, xylitol, maltitol, sorbitol, mannitol, lactitol, isomalt, erythritol, polyglycitol, polyglucitol, glycerol and stevia, and artificial sweeteners such as aspartame, acesulfame-κ, neotame, saccharine, and sucralose. The use of non-cariogenic sweeteners is preferred.

Thickening agents which may be present in the aqueous phase include other hydrocolloids such as starch, modified starch, xanthan, galactomannans (e.g. guar gum and locust bean gum), gum karaya, gum tragacanth, gum arabic, and cellulose derivatives (e.g. methylcelullose (MC), hydroxypropyl methylcellulose (HPMC), and carboxymethyl cellulose (CMC)), and any combination thereof. The thickening effect of such materials depends on the type of hydrocolloid used and its concentration, the other components and the pH of the formulation, etc. but suitable amounts may readily be determined.

Emulsifying agents may also be present in the aqueous phase. Suitable agents are well known for use in the food industry and in pharmaceutical and nutraceutical products. Any known emulsifying agent or agents may be selected for use in the emulsions according to the invention having in mind the need for compatibility with the remaining components of the emulsion, and the requirement that they should be suitable for oral delivery. Non-limiting examples of suitable emulsifying agents include proteins, for example soy protein, and amphiphilic polysaccharides such as HM pectin. Gum arabic, which is a mixture of glycoproteins and polysaccharides, may also be used. Other emulsifying agents suitable for use in the gelled emulsions include lecithins, monoglycerides, polysorbates (e.g. Polysorbate 65, Polysorbate 80, Polysorbate 20), Cremophor EL, Solutol HS15, poloxamers (e.g. Poloxamer 407, Poloxamer 188), and cellulose derivatives (e.g. methylcelullose (MC), hydroxypropyl methylcellulose (HPMC)).

Flavouring agents may be present in the compositions and may, for example, aid in taste masking certain lipids such as those which contain omega-3 fatty acids. Suitable flavours include, but are not limited to, citrus flavours, for example orange or lemon oil.

Suitable buffering agents are well known in the art and include, for example, sodium citrate, malic acid, etc. The pH of the aqueous phase of the emulsion may be adjusted to be in the range 2 to 9, particularly 3 to 7.5, e.g. 4 to 7.

In one set of embodiments, the gelled oil-in-water emulsion may additionally contain an oromucosal uptake enhancer in order to aid delivery of the cannabinoid active materials in the oral cavity, whether via sublingual or buccal delivery. The term "oromucosal uptake enhancer" refers to any substance which enhances uptake of the active materials via an oromucosal surface, e.g. via the buccal and/or sublingual surfaces in the mouth. This substance may, but need not necessarily be, an oromucosal adsorption enhancer, such as a mucoadhesive agent. The term "mucoadhesive agent" denotes a substance which exhibits an affinity for a mucosa surface, i.e. adheres to that surface through the formation of bonds which are generally non-covalent in nature. The oromucosal uptake enhancer will typically be provided in the aqueous phase.

Mucoadhesives suitable for use in the gelled emulsions herein described are well known in the art and may readily be selected. Non-limiting examples include natural materials such as agarose, chitosan, gelatin, hyaluronic acid, and various gums (e.g. guar, hakea, xanthan, gellan, carragenan, pectin and sodium alginate gums), as well as synthetic materials such as cellulose derivatives, and poly(acrylic acid) based polymers.

Other materials suitable for use as oromucosal uptake enhancers are known in the art and may readily be selected. As will be understood, such substances should be compatible with the remaining components of the emulsion and be suitable for oral delivery (i.e. they should be safe, non-toxic, non-irritating and non-allergenic). Ideally, they will be both pharmacologically and chemically inert and, in particular, they will provide an acceptable taste. Such substances may act in various different ways, for example these may enhance adsorption over the oromucosal membrane by altering the mucous rheology, by increasing the fluidity of the lipid bilayer membrane, by acting on components at tight junctions, and/or by overcoming the enzymatic barrier.

Suitable oromucosal uptake enhancers include, for example, surface penetration enhancers, such as surfactants (e.g. anionic surfactants such as sodium lauryl sulphate; cationic surfactants such as cetyl pyridinium chloride; and non-ionic surfactants such as poloxamers; Brij, Span, Myrj, Tween, etc.); cyclodextrins (including alpha, beta, and gamma cyclodextrins, and methylated beta-cyclodextrins); chelators (e.g. EDTA, citric acid, sodium salicylate, methoxy salicylates); terpenes (which may be present in essential oils, e.g. in peppermint oil (menthol)); bile salts (e.g. sodium glycol deoxycholate, sodium glycocholate, sodium tauro deoxycholate, and sodium tauro cholate); positively charged polymers (e.g. chitosan, trimethyl chitosan, polyacrylic acid); cationic compounds/amino acids (e.g. poly-L-arginine); mono- or polyols (e.g. ethanol, isopropanol, propylene glycol, polyethylene glycol, glycerol, and propanediol); sulfoxides (e.g. dimethyl sulfoxide (DMSO)); urea and its derivatives, and any combinations thereof. In one embodiment, the oromucosal uptake enhancer may be selected from citric acid, glycerol, menthol and any combination of these agents.

Fatty acids can also enhance uptake over the oromucosal membrane. Uptake enhancement will vary with fatty acid length and concentration. Medium chain length fatty acids are considered to be particularly suitable. For longer chain length fatty acids, those which are unsaturated are considered particularly beneficial. Examples of fatty acids which may be present to enhance uptake across the oromucosal membranes include oleic acid, caprylic acid, lauric acid, lyso phosphatidyl choline, and phosphatidyl choline. Lecithin may be used as a source of phosphatidyl choline, for example.

Particularly suitable for use as oromucosal uptake enhancers in the invention are cationic polymers such as chitosan and chitosan derivatives, particularly acetylated chitosan. Acetylated chitosans having a degree of acetylation in the range of from 30 to 60% may be preferred.

Where present, any oromucosal uptake enhancer may be present in an amount of from 0.1 to 2 wt.% based on the total weight of the composition.

In addition to the lipid(s) and active cannabinoid agent(s), the oil phase of the emulsion may also if desired contain physiologically tolerable lipid soluble materials, for example antioxidants (e.g. vitamin E), flavorings, and colors.

In some embodiments, other physiologically active agents may also be present in the gelled emulsions herein described. These may be provided in the aqueous and/or oil phases and may be dissolved and/or dispersed in one or both of these phases. Other actives which may be present in the oil phase include fat soluble active agents.

Examples of other physiologically active agents that may be included in the compositions of the invention include, for example, anti-inflammatory agents, such as NSAIDs (e.g. diclofenac), vitamin D, melatonin and magnesium (e.g. magnesium carbonate).

In one embodiment, the gelled oil-in-water emulsions according to the invention may comprise, consist essentially of, or consist of, the following components:
(a) water;
(b) one or more physiologically tolerable lipids;
(c) at least one cannabinoid;
(d) one or more physiologically tolerable gelling agents;
(e) one or more bulking agents;
(f) one or more buffering agents;
(g) optionally one or more viscosity modifying agents (e.g. thickening agents or plasticisers);
(h) optionally one or more oromucosal uptake enhancers; and
(i) optionally one or more additional physiologically active agents.

By "consisting essentially of" it is intended that the emulsions will be substantially free from (e.g. free from) other components which materially affect their properties. By "consists of" it is intended that the emulsions will be substantially free (e.g. free from) from any other components than those listed.

The compositions of the invention are provided in the form of a dose unit. By "dose unit" it is intended that the composition will be taken orally by the subject (e.g. administered to a patient) "as received", i.e. it will not be broken or cut before oral delivery. The weight of the dose unit will therefore be such that the composition is suitable for delivery in this way. For example, it may have an overall weight in the range from 50 to 3,000 mg, e.g. 250 to 3,000 mg or 500 to 2,500 mg, especially 100 to 2,000 mg, e.g. 750 to 2,000 mg, particularly 100 to 1,500 mg, more particularly 400 to 1,500 mg, more especially 400 to 1,000 mg.

In one set of embodiments, the dose units will generally be quite large, e.g. having a mass of from 400 to 3,000 mg, e.g. 600 to 1,500 mg. The overall dose unit weight may be selected as required. For example, it may be scaled up or down dependent on the nature of the selected active components and their intended dose, for example it may be determined according to the desired dosage of cannabinoid(s).

Each dose unit will contain one "core" of gelled oil-in-water emulsion as herein described. Each core may be formed from a larger piece of gelled emulsion which is divided, e.g. by cutting. More typically, however, each core will be formed by extrusion or moulding of a dose unit from a liquid emulsion, or incompletely gelled emulsion, prior to gelation (i.e. above the gelling temperature of the selected gelling agent or prior to the addition of any gelling initiator).

The compositions of the invention will typically consist of a core of the gelled oil-in-water emulsion. As will be understood, in this case the core will contain only the defined oil and aqueous phases, i.e. it will be free from any other components. However, as will be discussed, in other embodiments the core may be provided with a coating of a physiologically tolerable coating material. Such coatings may be of the type conventional in the pharmaceutical and nutraceutical industry and may be applied by any conventional means, for example by dipping or spraying.

In one set of embodiments, the gelled oil-in-water emulsions herein described may be provided with a coating. For example, these may be provided within a capsule shell which dissolves in the mouth. Viewed from another aspect the invention thus provides an orally administrable capsule comprising a capsule shell enclosing a gelled oil-in-water emulsion as herein described.

In the capsules of the invention, the shell may be of any physiologically tolerable material but will typically be a sugar, a biopolymer or a synthetic or semi-synthetic polymer which is soluble or disintegrable in saliva or fluid within the gastrointestinal tract. The shell may be soft, but is preferably substantially rigid. Particularly desirably, the capsules will have the consistency of a "jelly bean". The shell will preferably be of a material and a thickness to prevent oxidation of the contents. The shell may comprise a sugar, gelatin or cellulose, particularly a sugar or gelatin, for example sorbitol or gelatin. The use of sugars, gelatin and cellulose as capsule shell materials is well-known in the pharmaceutical and nutraceutical fields. Gelatin may be obtained from any of the sources herein described, including non-mammalian sources.

The capsule shell material may thus typically be a sugar, e.g. sucrose, fructose, maltose, xylitol, maltitol or sorbitol, but may additionally contain hydrocolloid materials such as for example gelatin, carageenan, alginate, pectin, cellulose, modified cellulose, starch, modified starch, gum arabic, etc. The capsule shell may contain other ingredients such as, for example, artificial sweeteners, colors, fillers, flavors, antioxidants, etc.

The capsule shell may be pre-formed such that the oil-in-water emulsion can be filled into the shell either as a liquid, or once set. Alternatively a shell precursor (e.g. a solution) may be coated onto the set emulsion, for example using standard coating techniques. If desired the capsule may be further coated, e.g. with a wax.

Preparation of the gelled oil-in-water emulsions herein described may be carried out by emulsification of the aqueous and oil phase components. It will be understood that emulsification is carried out under conditions in which the aqueous phase is a liquid (for example a viscous liquid), i.e. prior to the formation of a gel. Depending on the type of gelling agent selected, this will dictate the emulsifying conditions. For example, where the gelling agent undergoes a sol-gel transition, emulsification will be carried out at a temperature above the sol-gel transition temperature.

Subsequent cooling of the emulsion below the sol-gel temperature results in the desired gelled emulsion.

Prior to emulsification, the chosen cannabinoid(s) may be added to the oil phase. This may be done by dissolving the cannabinoid in the selected oil. CBD, for example, has a melting point of 67.5°C and is a crystalline solid at ambient temperature. Dissolution of the CBD in the oil phase without heating ensures there is no undesirable degradation of the active. Alternatively, the selected cannabinoid(s) may be added to a mixture of the aqueous and oil phase components prior to emulsification. During the emulsification process, the lipophilic cannabinoid material will typically migrate to the oil phase.

Emulsion formation may be effected by conventional techniques and using known equipment, for example a homogenizer based on the rotor-stator principle. The speed and duration of stirring may be adjusted as required, for example it may be varied to achieve the desired shearing force to provide the desired droplet size.

Emulsification will generally be carried out under a controlled atmosphere in order to avoid oxidative degradation of the lipid and/or the active cannabinoid materials. For example, emulsification may be carried out in the presence of a non-oxidising gas such as nitrogen. De-gassing to remove air bubbles may also be carried during the production process, for example prior to mixing the components of the emulsion, once the liquid emulsion has been formed, prior to packaging of the set emulsion, etc. De-gassing may be carried out using any conventional means such as the application of a vacuum, or sparging with a non-oxidising gas (e.g. nitrogen). After emulsification and gelling, the emulsion may be dried to reduce the water content. If dried, however, it will still retain a continuous gelled aqueous phase as herein described and a water content within the limits herein defined.

The gelled oil-in-water emulsions are provided in dose unit form as herein described. Individual dose units may be formed by methods such as molding, extrusion or cutting. Typically, however, the dose units may be formed by filling of the liquid emulsion into molds, e.g. the individual molds of a blister pack which is then sealed. The dose units will preferably be in tablet or lozenge form. However, for children they may conveniently be presented in a form which appeals to children, e.g. geometric shapes such as rods, strips and tubes, or animal, doll, or vehicle shapes.

In one set of embodiments, the cores may be shaped in a form which encourages retention of the composition in the mouth and so aids in oromucosal (e.g. sublingual or buccal) delivery of the cannabinoid(s). Disc-shaped cores are suitable for this purpose. Rod-shaped cores may also be suitable for placing under the tongue where sublingual delivery is desirable.

Methods for preparation of the gelled oil-in-water emulsions herein described form a further aspect of the invention. Viewed from a further aspect, the invention thus provides a method for preparing an orally administrable, gelled oil-in-water emulsion in unit dose form as herein described, said method comprising: forming an oil phase which comprises one or more physiologically tolerable lipids and at least one active cannabinoid; forming an aqueous phase comprising a physiologically tolerable gelling agent; combining said oil phase and said aqueous phase to form an oil-in-water emulsion; and allowing said emulsion to gel. Prior to or after allowing the emulsion to gel, the emulsion is divided into individual dose units.

The dose units are preferably individually packaged in air-tight containers, e.g. a sealed wrapper or more preferably a blister of a blister pack. In another aspect, the invention thus provides a package comprising an air-tight and light-tight compartment containing one dose unit of a composition according to the invention. By excluding both air (i.e. oxygen) and light from the packaged dose unit, long term stability of the active cannabinoid material(s) is enhanced.

The packages according to the invention are preferably provided in the form of blister packs containing at least two dose units, e.g. 2 to 100, preferably 6 to 30 dose units. The blister pack will generally comprise a metal or metal/plastic laminate sheet base having molded indentations in which the dosage form is placed. The pack is normally sealed with a foil, generally a metal or a metal/plastic laminate foil, for example by heating the areas between the indentations. The use of a metal or metal/plastic laminate to form the blister pack serves to prevent air (i.e. oxygen), light and humidity from penetrating the contents of the blister pack thus enhancing the stability of the cannabinoid material(s).

The packages according to the invention are preferably filled under a non-oxidising gas atmosphere (e.g. nitrogen) or are flushed with such a gas before sealing.

The gelled oil-in-water emulsions according to the invention find use both as pharmaceuticals, i.e. for therapeutic purposes, and as non-therapeutic nutraceuticals. The gelled form of the compositions herein described, and their good organoleptic characteristics (i.e. mouthfeel and taste), makes these particularly suitable for retention in the mouth without immediate swallowing. For example, the product may be held under the tongue (for sub-lingual delivery) or within the cheek cavity (for buccal delivery). The precise timing for retention in the mouth prior to dissolution will depend on the particular formulation but it is envisaged, for example, that the product may be held in the mouth for up to several minutes, e.g. from 1 to 15 minutes, prior to complete dissolution. On sucking, the gelled emulsion slowly melts and the active agents are delivered via the mouth cavity directly into the circulatory system. Sublingual or buccal delivery of the cannabinoid(s) minimises the first-pass metabolism effect.

When used as non-therapeutic nutraceuticals, for example, the compositions herein described may be used as a supplement (e.g. as a dietary supplement) for maintaining the general health and/or well-being of a subject. Examples of non-therapeutic nutraceutical uses include, but are not limited to, reduction in symptoms of anxiety, reduction in menopause symptoms, treatment of insomnia, reduction in menstrual symptoms (e.g. menstrual cramps), prevention of nausea, stimulating appetite, and use as a muscle relaxant. Other non-therapeutic nutraceutical uses include use for recreational purposes, for example in enhancing sexual pleasure.

The gelled oil-in-water emulsions herein described find use in the treatment or prevention of a range of medical conditions which are responsive to the chosen cannabinoid agent(s). As will be appreciated, the nature of such conditions will be dependent on the selected cannabinoid (and, where present, any other drug substance), but can readily be determined by those skilled in the art. For example, the gelled emulsions may be used to treat or prevent any of the following conditions: pain (e.g. cancer pain, arthritis pain), inflammation, neurological disorders, mood disorders (e.g. anxiety), epilepsy, sleep disorders, the symptoms of multiple sclerosis, anorexia (i.e. to enhance appetite), schizophrenia, inflammatory bowel disease (i.e. Crohn's disease and ulcerative colitis), and chemotherapy-induced nausea. More particularly the gelled oil-in-water emulsions may be used in the treatment of chronic pain (e.g. musculoskeletal pain) and chronic inflammatory diseases (e.g. rheumatoid arthritis and osteoarthritis).

Viewed from another aspect, the invention thus provides a gelled oil-in-water emulsion as herein described for oral use in therapy.

Viewed from still another aspect, the invention provides a gelled oil-in-water emulsion as herein described for oral use in the treatment of a condition responsive to a cannabinoid, wherein said condition is selected from pain (e.g. cancer pain, arthritis pain), inflammation, neurological disorders, mood disorders (e.g. anxiety), epilepsy, sleep disorders, the symptoms of multiple sclerosis, anorexia, schizophrenia, inflammatory bowel disease (e.g. Crohn's disease and ulcerative colitis), and chemotherapy-induced nausea.

In another aspect the invention provides the non-therapeutic use of a gelled oil-in-water emulsion as herein described as a nutraceutical.

The invention will now be described further with reference to the following non-limiting Examples and the accompanying figures in which:
- Figure 1:: Droplet size distribution for the emulsions prepared in Examples 24 to 27.
- Figure 2:: Droplet size distribution for the emulsions of Examples 24 and 27. These have the same composition but differ only in mixing speed used in their preparation.
- Figure 3:: Influence of mixing speed of the Ultra-turrax^{®} unit on the average volume-based (D[4,3]) droplet size for the emulsions in Examples 24 to 27.

### Examples

### Materials:

Unless otherwise specified, the gelatin is a Type A or Type B gelatin. Unless otherwise specified, it may have a bloom value of 150.

The MCT oil is based on coconut oil from *Cocos nucifera* supplied Henry Lamotte Oils GmbH, and has the following fatty acid composition (% given by weight):

| | | |
|---|---|---|
| Caproic acid | (C 6:0) | 0.1% |
| Caprylic acid | (C 8:0) | 55.0% |
| Capric acid | (C 10:0) | 44.8% |
| Lauric acid | (C 12:0) | 0.1% |
| Myristic acid | (C 14:0) | 0.0% |

"Hemp oil" is CBD standardised hemp oil containing 3.832 wt.% CBD, 99.5% pure by HPLC (THC content < 0.25) supplied by Hempro Int. GmbH & Co. KG.

### Examples 1 to 4 - Gelled oil-in-water emulsions containing CBD

Gelled oil-in-water emulsions having the compositions set out in Table 1 were made by adding the gelatin to the water and heating the solution to 70°C with stirring for 10 minutes. The sorbitol and xylitol were added stepwise to the mixture. , followed by the sodium citrate. Heating and stirring was continued between each addition to ensure full dissolution and the temperature was maintained between 50-75°C. After approx. 15 minutes, the resultant solution was degassed under vacuum to remove air bubbles. At a temperature of about 55°C the malic acid was added and dissolved in the mixture. Colour and flavouring agents and the oil containing the CBD was added to the resulting aqueous phase with stirring and homogenized into the mixture using a commercially available Ultra-Turrax^{®} unit. The resulting emulsion was once again degassed under vacuum to remove air bubbles. Whilst still liquid, the warm emulsion was filled into pre-formed cavities in a suitable packing material, sealed and allowed to set to form a gel at room temperature.

**Table 1: Composition of gelled oil-in-water emulsions containing CBD**

| | | **Example 1** | **Example 2** | **Example 3** | **Example 4** |
|---|---|---|---|---|---|
| Dose size (mg) | | 1500 | 1500 | 1500 | 1500 |
| | | wt.% | wt.% | wt.% | wt.% |
| Oil | | | | | |
| | Hemp oil | 16.6 | 16.6 | | |
| | Sunflower oil | 16.6 | 10 | 25.867 | 25.867 |

| Active/dose | | | | | |
|---|---|---|---|---|---|
| | CBD | | | 0.733 | 0.733 |
| | mg/dose | 9.54 | 9.54 | 10 | 10 |
| Water | | 18.6 | 20.6 | 20.6 | 20.6 |
| Gelatin | | | | | |
| | Bovine type B | 7.07 | 7.4 | 7.4 | 7.4 |
| | Fish 195 bloom type A | | | | |

| Sugar alcohols | | | | | |
|---|---|---|---|---|---|
| | Sorbitol | 11.01 | 12.5 | 12.5 | 12.5 |
| | Xylitol | 26 | 28.78 | 28.68 | 28.73 |

| Buffer salts | | | | | |
|---|---|---|---|---|---|
| | Sodium citrate | 1.8 | 1.8 | 1.8 | 1.8 |
| | Malic acid | 0.92 | 0.92 | 0.92 | 0.92 |
| Colour | | | | | |
| | Betacarotene | 0.5 | 0.5 | 0.5 | 0.5 |
| Flavour | | | | | |
| | Lemon | 0.9 | 0.9 | | 0.9 |
| | Lemon/Lime | | | 0.9 | |
| | Peppermint | | | 0.1 | 0.05 |
| **Total** | | **100** | **100** | **100** | **100** |
| | | | | | |
| Mixing conditions | | "Normal" | "Normal" | "Normal" | "Normal" |
| Ultra-turrax^{®} setting/minutes | | 2/7 | 2/7 | 2/7 | 2/7 |

### Example 5 - Gelled oil-in-water emulsion with 10 wt.% oil phase

Example 5 does not form part of the invention.
Water: 25.35 wt.%
Gelatin: 9 wt.%
Sorbitol: 15 wt.%
Xylitol: 36.57 wt.%
Trisodium citrate: 2.7 wt.%
Malic acid: 1.38 wt.%
MCT oil containing 10 mg CBD: 10 wt.%

The gelatin is added to the water and the solution is then heated to about 70°C with stirring for 10 minutes. The sorbitol and xylitol are then added stepwise to the mixture, followed by the trisodium citrate. Heating and stirring is continued between each addition to ensure full dissolution and the temperature is maintained between 50-75°C. The resultant solution is degassed under vacuum to remove air bubbles. At a temperature of about 55°C the malic acid is added and dissolved in the mixture.

The MCT oil containing the CBD is added to the resulting aqueous phase with stirring, and homogenized into the mixture using a commercially available Ultra-Turrax unit. The resulting emulsion is once again degassed under vacuum to remove air bubbles.

Whilst still liquid, the warm emulsion is filled into pre-formed cavities in a suitable packing material, sealed and allowed to set to form a gel at room temperature.

### Example 6 - Gelled oil-in-water emulsion with 20 wt.% oil phase

A gelled oil-in-water emulsion with the following composition is prepared analogously to Example 5:
Water: 22.53 wt.%
Gelatin: 8 wt.%
Sorbitol: 13.33 wt.%
Xylitol: 32.51 wt.%
Trisodium citrate: 2.4 wt.%
Malic acid: 1.23 wt.%
MCT oil containing 10 mg CBD: 20 wt.%

Example 7 - Gelled oil-in-water emulsion with 30 wt.% oil phase

A gelled oil-in-water emulsion with the following composition is prepared analogously to Example 5:
Water: 19.72 wt.%
Gelatin: 7 wt.%
Sorbitol: 11.67 wt.%
Xylitol: 28.44 wt.%
Trisodium citrate: 2.1 wt.%
Malic acid: 1.07 wt.%
MCT oil containing 10 mg CBD: 30 wt.%

### Example 8 - Gelled oil-in-water emulsion with 40 wt.% oil phase

A gelled oil-in-water emulsion with the following composition is prepared analogously to Example 5:
Water: 16.9 wt.%
Gelatin: 6 wt.%
Sorbitol: 10 wt.%
Xylitol: 24.38 wt.%
Trisodium citrate: 1.8 wt.%
Malic acid: 0.92 wt.%
MCT oil containing 10 mg CBD: 40 wt.%

### Example 9 - Gelled oil-in-water emulsion with 50 wt.% oil phase

A gelled oil-in-water emulsion with the following composition is prepared analogously to Example 5:
Water: 14.08 wt.%
Gelatin: 5 wt.%
Sorbitol: 8.33 wt.%
Xylitol: 20.32 wt.%
Trisodium citrate: 1.5 wt.%
Malic acid: 0.77 wt.%
MCT oil containing 10 mg CBD: 50 wt.%

### Example 10 - Gelled oil-in-water emulsion with 50 wt.% oil phase and higher loading of CBD

Gelled oil-in-water emulsions with the following composition may be prepared analogously to Example 5:
Water: 14.08 wt.%
Gelatin: 5 wt.%
Sorbitol: 8.33 wt.%
Xylitol: 20.32 wt.%
Trisodium citrate: 1.5 wt.%
Malic acid: 0.77 wt.%
MCT oil containing 20 - 300 mg CBD¹: 50 wt.%

¹The amount of CBD may be varied between 20 to 300 mg depending on the intended use of the composition.

### Example 11 - Gelled oil-in-water emulsion with 20 wt.% oil phase and a mucoadhesive agent

Gelled oil-in-water emulsions with the following composition may be prepared analogously to Example 5 except for the following initial steps: the chitosan is first dissolved in the weakly acidified water (water plus acetic acid) and, when completely dissolved, the alkaline buffering agent (typically phosphate) is added. The gelatin is then added to the resulting aqueous solution and the temperature is raised to 70°C with stirring for 10 minutes. The remainder of the procedure from Example 5 is then followed.
Water: 22.53 wt.%
Gelatin: 8 wt.%
Sorbitol: 13.33 wt.%
Xylitol: 32.51 wt.%
MCT oil containing 20 - 300 mg CBD¹: 20 wt.%
Acetylated chitosan (mucoadhesive agent): 1 wt.%
Acetic acid: as required
Alkaline buffering agent (pH 7.5): 2.63 wt.%

¹The amount of CBD may be varied between 20 to 300 mg depending on the intended use of the composition.

### Example 12 - Gelled oil-in-water emulsion containing other oromucosal uptake enhancing agents

A gelled oil-in-water emulsion having the composition set out in Table 2 may be prepared analogously to Example 5, subject to the following modifications: glycerol is added after the xylitol/sorbitol; citric acid and peppermint essential oil are added before the oil and dissolved; and the lechitin is added towards the end of the homogenisation step. A cannabinoid (e.g. CBD) may be included as part of the oil phase.

**Table 2: Composition of gelled oil-in-water emulsions**

| | | wt.% |
|---|---|---|
| Oil | | |
| | MCT | 47 |
| Water | | 18 |
| Gelatin | | |
| | Bovine type B | 5.5 |

| Sugar alcohols | | |
|---|---|---|
| | Sorbitol | 6.95 |
| | Xylitol | 16.855 |

| Buffer salts | | |
|---|---|---|
| | Sodium citrate | 2.025 |

| Oromucosal uptake enhancing agents | | |
|---|---|---|
| | Citric acid | 1 |
| | Peppermint essential oil, 40% Menthol | 0.5 |
| | Glycerol | 2 |
| | Lecithin, 95% | 0.1 |

| Colour | | |
|---|---|---|
| | Betacarotene 30% | 0.07 |
| **Total** | | **100** |

### Example 13 - Gelled oil-in-water emulsion with 20 wt.% oil phase containing CBD + Dronabinol

Gelled oil-in-water emulsions with the following composition may be prepared analogously to Example 5. The Dronabinol is added to the oil phase together with the CBD.
Water: 22.53 wt.%
Gelatin: 8 wt.%
Sorbitol: 13.33 wt.%
Xylitol: 32.51 wt.%
Trisodium citrate: 2.4 wt.%
Malic acid: 1.23 wt.%
MCT oil containing 20 - 300 mg CBD¹ and 2.5 - 10 mg Dronabinol²: 20 wt.%

¹The amount of CBD may be varied between 20 to 300 mg depending on the intended use of the composition.

²The amount of Dronabinol may be varied between 2.5 to 10 mg depending on the intended use of the composition.

### Example 14 - Gelled oil-in-water emulsion with 20 wt.% oil phase containing CBD + NSAID (Diclofenac)

Gelled oil-in-water emulsions with the following composition may be prepared analogously to Example 5. Diclofenac may be added to the aqueous phase or to the oil phase prior to mixing of these phases and homogenization.
Water: 21 wt.%
Gelatin: 8 wt.%
Sorbitol: 13 wt.%
Xylitol: 31.05 wt.%
Trisodium citrate: 2.4 wt.%
Malic acid: 1.22 wt.%
MCT oil containing 20 - 300 mg CBD¹: 20 wt.%
Diclofenac (50 mg): 3.33 wt.%

¹The amount of CBD may be varied between 20 to 300 mg depending on the intended use of the composition.

### Example 15 - Gelled oil-in-water emulsion with 20 wt.% oil phase containing CBD + other active agents

Gelled oil-in-water emulsions based on MCT oil and including CBD plus any of vitamin D, melatonin (0.5 to 5 mg) and magnesium carbonate (40 mg - approx. 10% RDA) may be prepared analogously to Example 14. The gelled emulsions can be used as nutraceuticals.

### Example 16 - Gelled oil-in-water emulsion with agar as gelling agent

Gelled oil-in-water emulsions with the following composition may be prepared analogously to Example 5 except for the following initial steps: agar is added to the water and the solution is then heated to about 90°C with stirring for 30 minutes or until fully dissolved. The temperature is then lowered to 70°C and the procedure from Example 5 is followed in which the citric acid ester (emulsifying agent) is added together with the sugar alcohols.
Water: 26 wt.%
Agar: 2 wt.%
Citric acid ester (Grinsted Citrem N 12): 1 wt.%
Sorbitol: 14 wt.%
Xylitol: 28 wt.%
Trisodium citrate: 2.4 wt.%
Malic acid: 1.6 wt.%
MCT oil containing 10 mg CBD: 25 wt.%

### Example 17 - Gelled oil-in-water emulsion with agar as gelling agent

Gelled oil-in-water emulsions with the following composition may be prepared analogously to Example 5 except for the following initial steps: agar and locust bean gum are added to the water and the solution is then heated to about 90°C with stirring for 30 minutes or until fully dissolved. The temperature is then lowered to 70°C and the procedure from Example 5 is followed in which the citric acid ester is added together with the sugar alcohols.
Water: 26 wt.%
Agar: 1.8 wt.%
Citric acid ester (Grinsted Citrem N 12): 1 wt.%
Locust Bean Gum: 0.2 wt.%
Sorbitol: 14 wt.%
Xylitol: 28 wt.%
Trisodium citrate: 2.4 wt.%
Malic acid: 1.6 wt.%
MCT oil containing 10 mg CBD: 25 wt.%

### Example 18 - Gelled oil-in-water emulsion with κ-Carrageenan as gelling agent

Gelled oil-in-water emulsions with the following composition may be prepared analogously to Example 5 except for the following initial steps: κ-Carrageenan is added to the water and the solution is heated to about 80°C with stirring for 15 minutes or until fully dissolved. The temperature is then lowered to 70°C and the procedure from Example 5 is followed in which the soy protein and potassium salt are added together with the sugar alcohols.
Water: 26 wt.%
κ-Carrageenan: 1.5 wt.%
KCI: 0.4 wt.%
Citric acid ester (Grinsted Citrem N 12): 1 wt.%
Sorbitol: 14 wt.%
Xylitol: 28 wt.%
Trisodium citrate: 2.5 wt.%
Malic acid: 1.6 wt.%
MCT oil containing 10 mg CBD: 25 wt.%

### Example 19 - Gelled oil-in-water emulsion with i-Carrageenan as gelling agent

Gelled oil-in-water emulsions with the following composition may be prepared analogously to Example 5 except for the following initial steps: i-Carrageenan is added to the water and the solution is heated to about 80°C with stirring for 15 minutes or until fully dissolved. The temperature is then lowered to 70°C and the procedure from Example 5 is followed in which the soy protein and calcium salt are added together with the sugar alcohols.
Water: 26 wt.%
i-Carrageenan: 1.5 wt.%
CaCl₂: 0.4 wt.%
Citric acid ester (Grinsted Citrem N 12): 1 wt.%
Sorbitol: 14 wt.%
Xylitol: 28 wt.%
Trisodium citrate: 2.5 wt.%
Malic acid: 1.6 wt.%
MCT oil containing 10 mg CBD: 25 wt.%

### Example 20 - Gelled oil-in-water emulsion with HM pectin as gelling agent

Water: 27 wt.%
HM Pectin: 1.5 wt.%
Sorbitol: 16.75 wt.%
Xylitol: 29.75 wt.%
50% citric acid solution: As required (dependent on required pH)
MCT oil containing 10 mg CBD: 25 wt.%
pH 3.0 - 3.1

A dry mix of HM pectin and sugar alcohols is prepared and dispersed in water. The resulting solution is brought to the boil under stirring until the pectin is fully dissolved. Still at a temperature ≥ 95°C, the MCT oil containing the CBD is added with stirring. The mixture is homogenized using a commercially available Ultra-Turrax unit to produce the emulsion. Citric acid is added as required to obtain the recommended pH range. Whilst still liquid, the warm emulsion is filled into pre-formed cavities in a suitable packing material, sealed and allowed to set to form a gel at room temperature.

### Example 21 - Gelled oil-in-water emulsion with LM pectin as gelling agent

Water: 26 wt.%
LM Pectin: 1.5 wt.%
Calcium citrate: 0.2 wt.%
Citric acid ester (Grinsted Citrem N 12): 1 wt.%
Trisodium citrate: 0.25 wt.%
Sorbitol: 16.75 wt.%
Xylitol: 29.3 wt.%
50% citric acid solution: As required (dependent on desired pH)
MCT oil containing 10 mg CBD: 25 wt.%
pH 3.4 - 3.7

A dry mix of LM pectin, sugar alcohols and calcium citrate is prepared and dispersed in water. The resulting solution is brought to the boil under stirring until the pectin is fully dissolved. Still at a temperature ≥ 95°C, the MCT oil containing the CBD is added with stirring. The mixture is homogenized using a commercially available Ultra-Turrax unit to produce the emulsion. Citric acid is added as required to obtain the recommended pH range. Whilst still liquid, the warm emulsion is filled into pre-formed cavities in a suitable packing material, sealed and allowed to set to form a gel at room temperature.

### Example 22 - Gelled oil-in-water emulsion with alginate as gelling agent

Water: 26 wt.%
Alginate: 1.5 wt.%
Calcium sulphate: 0.3 wt.%
Sodium pyrophosphate: 0.03 wt.%
Citric acid ester (Grinsted Citrem N 12): 1 wt.%
Sorbitol: 16.75 wt.%
Xylitol: 29.4 wt.%
MCT oil containing 10 mg CBD: 25 wt.%

A dry mix of alginate, sugar alcohols, soy protein and sodium pyrophosphate is prepared and dispersed in water at 20°C until dissolved. Still at room temperature, the MCT oil containing the CBD is added with stirring. The mixture is homogenized using a commercially available Ultra-Turrax unit to produce the emulsion. Calcium sulphate is then added under vigorous stirring. Whilst still liquid, the emulsion is filled into pre-formed cavities in a suitable packing material, sealed and allowed to set to form a gel at room temperature.

### Example 23 - Gelled oil-in-water emulsion with alginate as gelling agent

Water: 26 wt.%
Alginate: 1.5 wt.%
Calcium carbonate: 0.06 wt.%
Glucono-δ-lactone: 0.2 wt.%
Citric acid ester (Grinsted Citrem N 12): 1 wt.%
Sorbitol: 16.75 wt.%
Xylitol: 29.4 wt.%
MCT oil containing 10 mg CBD: 25 wt.%

A dry mix of alginate, sugar alcohols, soy protein and calcium carbonate is prepared and dispersed in water at 20°C. Still at room temperature, the MCT oil containing the CBD is added with stirring. The mixture is homogenized using a commercially available Ultra-Turrax unit to produce the emulsion. Glucono-δ-lactone is then added under vigorous stirring. Whilst still liquid, the emulsion is filled into pre-formed cavities in a suitable packing material, sealed and allowed to set to form a gel at room temperature.

### Examples 24-27 - Effect of mixing speed on oil droplet size

The compositions set out in Table 3 are made analogously to those of Examples 1 to 4.

**Table 3: Compositions of MCT-based emulsions**

| | | **Example 24** | **Example 25** | **Example 26** | **Example 27** |
|---|---|---|---|---|---|
| | | wt.% | wt.% | wt.% | wt.% |
| Oil | | | | | |
| | MCT | 25 | 40 | 55 | 25 |
| Water | | 19.1 | 17 | 16.7 | 19.1 |

| Gelatin | | | | | |
|---|---|---|---|---|---|
| | Bovine type B (2350) | | 6 | 4.7 | |
| | Fish 195 bloom type A (2751) | 6.2 | | | 6.2 |

| Sugar alcohols | | | | | |
|---|---|---|---|---|---|
| | Sorbitol | 14.5 | 10 | | 14.5 |
| | Xylitol | 29.56 | 22.86 | 16.25 | 29.56 |
| | Glycerine | | | 4 | |

| Buffer salts | | | | | |
|---|---|---|---|---|---|
| | Sodium citrate | 1.8 | 1.8 | 1.6 | 1.8 |
| | Citric acid | | | | |
| | Malic acid | 0.92 | 0.92 | 0.7 | 0.92 |

| Thickeners | | | | | |
|---|---|---|---|---|---|
| | Gum Arabic | 1.5 | | | 1.5 |

| Colour | | | | | |
|---|---|---|---|---|---|
| | Carmine | 0.05 | 0.05 | 0.05 | 0.05 |

| Flavour | | | | | |
|---|---|---|---|---|---|
| | Lemon/Lime | 1 | 1 | 0.7 | 1 |
| | Raspberry | 0.37 | 0.37 | 0.3 | 0.37 |
| **Total** | | **100** | **100** | **100** | **100** |
| | | | | | |
| Mixing conditions | | "Normal" | "Medium" | "Low" | "High" |
| Ultra-turrax^{®} setting/minutes | | 2/7 | 1.75/7 | 1.25/7 | 3.5/10 |
| aw | | 0.694 | | | |

Droplet sizes and size distributions were measured using a Malvern Mastersizer 3000 (Worcestershire, UK) connected to a Hydro MV, wet dispersion unit (Malvern,Worcestershire, UK). Analysis of the data was performed using the manufacturer's software (Mastersizer 3000, v1.0.1). Testing was carried out by dissolving and diluting the gelled emulsion in a 10% (v/v) HCl solution (1:100) at 50°C. The refractive index of water and corn oil was set to 1.33 (solvent) and 1.47 (dispersed phase), respectively, and the absorption index of the dispersed droplets set to 0.01. To avoid multiple scattering or low intensity of the scattered light, each dissolved emulsion was added to the dispersion unit (containing ~125 mL water), until an obscuration of approximately 10% was obtained.

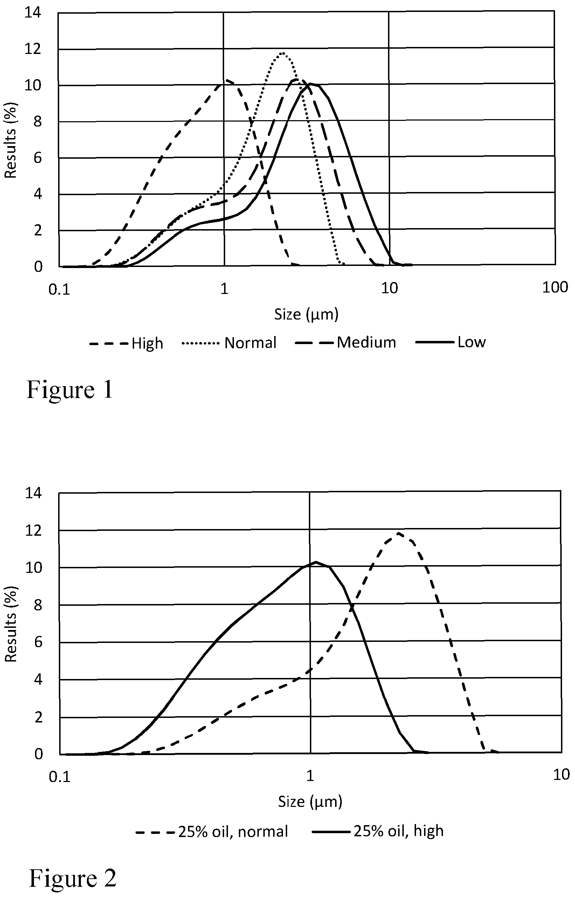

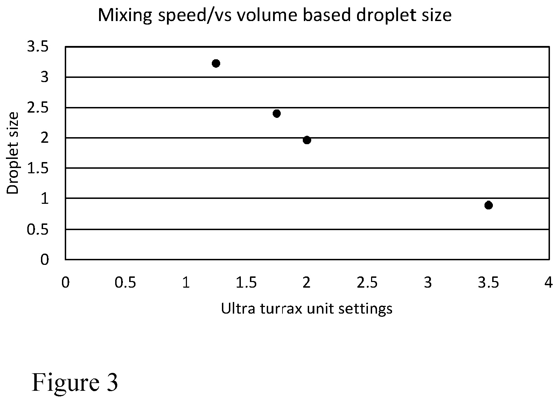

Droplet size distributions for the different emulsions are shown in Figures 1-3. Figure 1 shows the droplet size distributions achieved by varying the mixing speed: High - Normal - Medium - Low corresponds to the setting of the Ultra-turrax^{®} unit and mixing time given in table 3. Figure 2 compares the droplet size distribution results for the emulsions of Examples 24 and 27 which have the same composition and differ only in mixing speed. Figure 3 shows the influence of mixing speed of the Ultra-turrax^{®} unit on the average volume-based (D[4,3]) droplet size.

### Example 28 - packaging

### Blister packs:

Prior to setting, the emulsions produced in any of Examples 1 to 23 may be filled into blister trays made from a metal/plastic laminate over which a plastic/metal foil laminate is heat sealed.

### Strips:

Prior to setting, the emulsions produced in any of Examples 1 to 23 may be extruded into individual strips which, once set, are then sealed into individual plastic/metal foil laminate sachets. Alternatively, a single extruded strip, once set, may be cut into individual strips according to need prior to packaging.

### Example 29 - Coated gelled emulsions

The set emulsions produced in any of Examples 1 to 23 may be coated with a sorbitol solution comprising sorbitol (80 wt.%), lemon flavour (0.15 wt.%), yellow colour (0.5 wt.%) and water (ad 100 wt.%). The coating solution may be cured at 99-95°C for 4-5 hours before application. Coating is carried out by dipping or panning at 20-45°C. Several layers of coating material may be added with drying between each layer until the final composite layer is hard.

Alternatively, prior to setting, the liquid emulsion prepared in any of Examples 1 to 23 may be filled into soft capsule shells, for example commercially available gelatin capsule shells). This may be done using a conventional soft-gel machine. Typically such capsule shells comprise gelatin (40 wt.%), glycerol (30 wt.%), lemon flavour (0.15 wt.%), yellow colour (0.5 wt.%) and water (ad 100 wt.%).

## Claims

1. An orally administrable, gelled oil-in-water emulsion in unit dose form, wherein the emulsion is a self-supporting, viscoelastic solid having a gelled aqueous phase and an oil phase which comprises one or more physiologically tolerable lipids and at least one cannabinoid, and wherein the oil phase constitutes from 20 to 50 wt.% of the emulsion.

2. An orally administrable, gelled oil-in-water emulsion as claimed in claim 1, wherein said at least one cannabinoid is selected from tetrahydrocannabinols, tetrahydrocannabinolic acids, cannabidiol, cannabidiolic acid, cannabigerol, cannabigerolic acid, cannabigerovarin, cannabigerovarinic acid, cannabichromene, cannabichromenic acid, cannabidivarin, cannabidivarinic acid, cannabivarin, cannabivarinic acid, tetrahydrocannabivarin, tetrahydrocannabivarinic acid, cannabinol, cannabinolic acid, cannabinodiol, cannabielsoin, cannabicyclol, cannabicitran, isomers thereof, and mixtures thereof, preferably wherein said at least one cannabinoid is selected from a tetrahydrocannabinol (e.g. a delta-9-tetrahydrocannabinol) and/or a cannabidiol.

3. An orally administrable, gelled oil-in-water emulsion as claimed in claim 1 or claim 2, wherein said one or more physiologically tolerable lipids are derived from rapeseed oil, sunflower oil, corn oil, olive oil, sesame oil, palm kernel oil, coconut oil, a nut oil, or hemp oil.

4. An orally administrable, gelled oil-in-water emulsion as claimed in any one of the preceding claims, wherein said one or more physiologically tolerable lipids are fatty acids or derivatives thereof, e.g. the carboxylic esters, carboxylic anhydrides, glycerides (i.e. mono-, di-, or triglycerides) and phospholipids; preferably wherein said fatty acids or derivatives thereof have a saturated or unsaturated hydrocarbon chain comprising from 4 to 28 carbon atoms, preferably from 6 to 12 carbons.

5. An orally administrable, gelled oil-in-water emulsion as claimed in claim 4, wherein said fatty acids or derivatives thereof have a saturated hydrocarbon chain, preferably wherein said fatty acids or derivatives thereof have from 8 to 12, e.g. 8, 10 or 12, carbon atoms in the hydrocarbon chain; or wherein said fatty acids or derivatives thereof have an unsaturated hydrocarbon chain, preferably wherein said fatty acids are ω-3, ω-6 or ω-9 fatty acids, or derivatives thereof.

6. An orally administrable, gelled oil-in-water emulsion as claimed in claim 4 or claim 5, wherein said fatty acid derivatives are glycerides, preferably triglycerides; optionally wherein said fatty acid derivatives are medium-chain triglycerides having two or three medium-chain fatty acids which may be identical or different, preferably fatty acids having 6 to 12 carbon atoms, e.g. 8 or 10 carbons.

7. An orally administrable, gelled oil-in-water emulsion as claimed in any one of the preceding claims, wherein the oil phase is dispersed in the gelled aqueous phase in the form of oil droplets having a diameter in the range from 10 nm to 100µm, preferably wherein the oil droplets have an average diameter in the range from 100 nm to 1µm.

8. An orally administrable, gelled oil-in-water emulsion as claimed in any one of the preceding claims, wherein said aqueous phase comprises a physiologically tolerable gelling agent selected from alginates, pectins, carrageenans, gelatin, gellan gum and agar, preferably wherein the gelling agent is gelatin, optionally wherein the gelatin is present in the aqueous phase at a concentration of 5 to 25 wt.% based on the weight of the aqueous phase.

9. An orally administrable, gelled oil-in-water emulsion as claimed in any one of the preceding claims, wherein the aqueous phase further comprises one or more bulking agents, for example sugar alcohols, preferably wherein said bulking agents are present at a concentration of from 45 to 70 wt.%, preferably 50 to 65 wt.%, e.g. 55 to 60 wt.%, based on the aqueous phase.

10. An orally administrable, gelled oil-in-water emulsion as claimed in any one of the preceding claims which further comprises an oromucosal uptake enhancer, preferably wherein said oromucosal uptake enhancer is a cationic polymer, more preferably chitosan or a chitosan derivative.

11. A package comprising an air-tight and light-tight compartment containing one dose unit of the gelled oil-in-water emulsion as claimed in any one of the preceding claims.

12. A method for the preparation of an orally administrable, gelled oil-in-water emulsion in unit dose form as claimed in any one of claims 1 to 10, said method comprising the steps of:
(i) forming an oil phase which comprises one or more physiologically tolerable lipids and at least one cannabinoid; forming an aqueous phase comprising a physiologically tolerable gelling agent; combining said oil phase and said aqueous phase to form an oil-in-water emulsion; and allowing said emulsion to gel; or
(ii) forming an oil phase which comprises one or more physiologically tolerable lipids; forming an aqueous phase comprising a physiologically tolerable gelling agent; combining said oil phase, said aqueous phase and at least one cannabinoid to form an oil-in-water emulsion; and allowing said emulsion to gel.

13. A gelled oil-in-water emulsion as claimed in any one of claims 1 to 10 for oral use as a medicament or for oral use in therapy.

14. A gelled oil-in-water emulsion as claimed in any one of claims 1 to 10 for oral use in the treatment of a condition responsive to a cannabinoid, wherein said condition is selected from pain (e.g. cancer pain, arthritis pain), inflammation, neurological disorders, mood disorders (e.g. anxiety), epilepsy, sleep disorders, the symptoms of multiple sclerosis, anorexia, schizophrenia, inflammatory bowel disease (e.g. Crohn's disease and ulcerative colitis), and chemotherapy-induced nausea, preferably wherein said condition is selected from chronic pain (e.g. musculoskeletal pain) and chronic inflammatory diseases (e.g. rheumatoid arthritis and osteoarthritis).

15. Non-therapeutic use of a gelled oil-in-water emulsion as claimed in any one of claims 1 to 10 as a nutraceutical.

## Patentansprüche

1. Oral verabreichbare gelierte Öl-in-Wasser-Emulsion in Einheitsdosisform, wobei die Emulsion ein selbsttragender, viskoelastischer Festkörper ist, der eine gelierte wässrige Phase und eine Ölphase aufweist, die ein oder mehrere physiologisch verträgliche Lipide und mindestens ein Cannabinoid umfasst, und wobei die Ölphase von 20 bis 50 Gew.-% der Emulsion ausmacht.

2. Oral verabreichbare gelierte Öl-in-Wasser-Emulsion nach Anspruch 1, wobei das mindestens eine Cannabinoid aus Tetrahydrocannabinolen, Tetrahydrocannabinolsäuren, Cannabidiol, Cannabidiolsäure, Cannabigerol, Cannabigerolsäure, Cannabigerovarin, Cannabigerovarinsäure, Cannabichromen, Cannabichromensäure, Cannabidivarin, Cannabidivarinsäure, Cannabivarin, Cannabivarinicsäure, Tetrahydrocannabivarin, Tetrahydrocannabivarinsäure, Cannabinol, Cannabinolsäure, Cannabinodiol, Cannabielsoin, Cannabicyclol, Cannabicitran, Isomeren davon und Mischungen davon ausgewählt ist, wobei das mindestens eine Cannabinoid vorzugsweise aus einem Tetrahydrocannabinol (z. B. einem Delta-9-tetrahydrocannabinol) und/oder einem Cannabidiol ausgewählt ist.

3. Oral verabreichbare gelierte Öl-in-Wasser-Emulsion nach Anspruch 1 oder Anspruch 2, wobei das eine oder die mehreren physiologisch verträglichen Lipide von Rapsöl, Sonnenblumenöl, Maisöl, Olivenöl, Sesamöl, Palmkernöl, Kokosöl, einem Nussöl oder Hanföl abgeleitet sind.

4. Oral verabreichbare gelierte Öl-in-Wasser-Emulsion nach einem der vorstehenden Ansprüche, wobei das eine oder die mehreren physiologisch verträglichen Lipide Fettsäuren oder Derivate davon sind, z. B. Carbonsäureester, Carbonsäureanhydride, Glyceride (z. B. Mono-, Di- oder Triglyceride) und Phospholipide; wobei die Fettsäuren oder Derivate davon vorzugsweise eine gesättigte oder ungesättigte Kohlenwasserstoffkette aufweisen, die von 4 bis 28 Kohlenstoffatome, vorzugsweise von 6 bis 12 Kohlenstoffe umfassen.

5. Oral verabreichbare gelierte Öl-in-Wasser-Emulsion nach Anspruch 4, wobei die Fettsäuren oder Derivate davon eine gesättigte Kohlenwasserstoffkette aufweisen, wobei die Fettsäuren oder Derivate davon vorzugsweise von 8 bis 12, z. B. 8, 10 oder 12, Kohlenstoffatome in der Kohlenwasserstoffkette aufweisen; oder wobei die Fettsäuren oder Derivate davon eine ungesättigte Kohlenwasserstoffkette aufweisen, wobei die Fettsäuren vorzugsweise ω-3, ω-6 oder ω-9 Fettsäuren oder Derivate davon sind.

6. Oral verabreichbare gelierte Öl-in-Wasser-Emulsion nach Anspruch 4 oder Anspruch 5, wobei die Fettsäurederivate Glyceride, vorzugsweise Triglyceride sind; wobei optional die Fettsäurederivate mittelkettige Triglyceride sind, die zwei oder drei mittelkettige Fettsäuren aufweisen, die identisch oder unterschiedlich sein können, vorzugsweise Fettsäuren, die 6 bis 12 Kohlenstoffatome, z. B. 8 oder 10 Kohlenstoffe aufweisen.

7. Oral verabreichbare gelierte Öl-in-Wasser-Emulsion nach einem der vorstehenden Ansprüche, wobei die Ölphase in der gelierten wässrigen Phase in der Form von Öltröpfchen dispergiert ist, die einen Durchmesser im Bereich von 10 nm bis 100 µm aufweisen, wobei die Öltröpfchen vorzugsweise einen durchschnittlichen Durchmesser im Bereich von 100 nm bis 1 µm aufweisen.

8. Oral verabreichbare gelierte Öl-in-Wasser-Emulsion nach einem der vorstehenden Ansprüche, wobei die wässrige Phase ein physiologisch verträgliches Geliermittel umfasst, das aus Alginaten, Pektinen, Carrageenanen, Gelatine, Gellangummi und Agar ausgewählt ist, wobei das Geliermittel vorzugsweise Gelatine ist, wobei die Gelatine optional in der wässrigen Phase bei einer Konzentration von 5 bis 25 Gew.-% basierend auf dem Gewicht der wässrigen Phase vorliegt.

9. Oral verabreichbare gelierte Öl-in-Wasser-Emulsion nach einem der vorstehenden Ansprüche, wobei die wässrige Phase weiter einen oder mehrere Füllstoffe umfasst, zum Beispiel Zuckeralkohole, wobei die Füllstoffe vorzugsweise bei einer Konzentration von 45 bis 70 Gew.-%, vorzugsweise 50 bis 65 Gew.-%, z.B. 55 bis 60 Gew.-%, basierend auf der wässrigen Phase, vorliegen.

10. Oral verabreichbare gelierte Öl-in-Wasser-Emulsion nach einem der vorstehenden Ansprüche, die weiter einen oromukosalen Aufnahmeverstärker umfasst, wobei der oromukosale Aufnahmeverstärker vorzugsweise ein kationisches Polymer, noch bevorzugter Chitosan oder ein Chitosan-Derivat ist.

11. Packung, umfassend ein luftdichtes und lichtdichtes Abteil, das eine Dosiseinheit der gelierten Öl-in-Wasser-Emulsion nach einem der vorstehenden Ansprüche enthält.

12. Verfahren zum Zubereiten einer oral verabreichbaren gelierten Öl-in-Wasser-Emulsion in Einheitsdosisform nach einem der Ansprüche 1 bis 10, wobei das Verfahren die folgenden Schritte umfasst:
(i) Bilden einer Ölphase, die ein oder mehrere physiologisch verträgliche Lipide und mindestens ein Cannabinoid umfasst; Bilden einer wässrigen Phase, die ein physiologisch verträgliches Geliermittel umfasst; Kombinieren der Ölphase und der wässrigen Phase, um eine Öl-in-Wasser-Emulsion zu bilden; und Zulassen, dass die Emulsion geliert; oder
(ii) Bilden einer Ölphase, die ein oder mehrere physiologisch verträgliche Lipide umfasst; Bilden einer wässrigen Phase, die ein physiologisch verträgliches Geliermittel umfasst; Kombinieren der Ölphase, der wässrigen Phase und mindestens eines Cannabinoids, um eine Öl-in-Wasser-Emulsion zu bilden; und Zulassen, dass die Emulsion geliert.

13. Gelierte Öl-in-Wasser-Emulsion nach einem der Ansprüche 1 bis 10 zur oralen Verwendung als Medikament oder zur oralen Verwendung in einer Therapie.

14. Gelierte Öl-in-Wasser-Emulsion nach einem der Ansprüche 1 bis 10 zur oralen Verwendung in der Behandlung eines Zustands, der auf ein Cannabinoid anspricht, wobei der Zustand aus Schmerz (z. B. Krebsschmerz, Arthritisschmerz), Entzündung, neurologischen Störungen, Gemütsstörungen (z. B. Angst), Epilepsie, Schlafstörungen, den Symptomen von multipler Sklerose, Anorexie, Schizophrenie, entzündlicher Darmkrankheit (z. B. Crohn-Krankheit und Colitis ulcerosa) und durch Chemotherapie verursachte Nausea ausgewählt ist, wobei der Zustand vorzugsweise aus chronischem Schmerz (z. B. musculoskeletaler Schmerz) und chronischen Entzündungskrankheiten (z. B. rheumatoide Arthritis und Osteoarthritis) ausgewählt ist.

15. Nicht therapeutische Verwendung einer gelierten Öl-in-Wasser-Emulsion nach einem der Ansprüche 1 bis 10 als Nutraceutical.

## Revendications

1. Émulsion d'huile dans eau gélifiée administrable par voie orale sous forme de dose unitaire, dans laquelle l'émulsion est un solide viscoélastique autonome présentant une phase aqueuse gélifiée et une phase huileuse qui comprend un ou plusieurs lipides physiologiquement tolérables et au moins un cannabinoïde, et dans laquelle la phase huileuse constitue entre 20 % en poids et 50 % en poids de l'émulsion.

2. Émulsion d'huile dans eau gélifiée administrable par voie orale selon la revendication 1, dans laquelle ledit au moins un cannabinoïde est choisi parmi des tétrahydrocannabinols, des acides tétrahydrocannabinoliques, le cannabidiol, l'acide cannabidiolique, le cannabigérol, l'acide cannabigérolique, la cannabigérovarine, l'acide cannabigérovarinique, le cannabichromène, l'acide cannabichroménique, la cannabidivarine, l'acide cannabidivarinique, la cannabivarine, l'acide cannabivarinique, la tétrahydrocannabivarine, l'acide tétrahydrocannabivarinique, le cannabinol, l'acide cannabinolique, le cannabinodiol, le cannabielsoïne, le cannabicyclol, le cannabicitran, des isomères de ceux-ci, et des mélanges de ceux-ci, de préférence dans laquelle ledit au moins un cannabinoïde est choisi parmi un tétrahydrocannabinol (par exemple un delta-9-tétrahydrocannabinol) et/ou un cannabidiol.

3. Émulsion d'huile dans eau gélifiée administrable par voie orale selon la revendication 1 ou la revendication 2, dans laquelle ledit un ou lesdits plusieurs lipides physiologiquement tolérables sont dérivés d'huile de colza, d'huile de tournesol, d'huile de maïs, d'huile d'olive, d'huile de sésame, d'huile de palmiste, d'huile de coprah, d'une huile de noix, ou d'huile de chanvre.

4. Émulsion d'huile dans eau gélifiée administrable par voie orale selon l'une quelconque des revendications précédentes, dans laquelle ledit un ou lesdits plusieurs lipides physiologiquement tolérables sont des acides gras ou des dérivés de ceux-ci, par exemple les esters carboxyliques, anhydrides carboxyliques, glycérides (c'est-à-dire mono-, di-, ou triglycérides) et phospholipides ; de préférence dans laquelle lesdits acides gras ou dérivés de ceux-ci présentent une chaîne hydrocarbonée saturée ou insaturée comprenant de 4 à 28 atomes de carbone, de préférence de 6 à 12 atomes de carbone.

5. Émulsion d'huile dans eau gélifiée administrable par voie orale selon la revendication 4, dans laquelle lesdits acides gras ou dérivés de ceux-ci présentent une chaîne hydrocarbonée saturée, de préférence dans laquelle lesdits acides gras ou dérivés de ceux-ci présentent de 8 à 12, par exemple 8, 10 ou 12, atomes de carbone dans la chaîne hydrocarbonée ; ou dans laquelle lesdits acides gras ou dérivés de ceux-ci présentent une chaîne hydrocarbonée insaturée, de préférence dans laquelle lesdits acides gras sont des acides gras ω-3, ω-6 ou ω-9, ou des dérivés de ceux-ci.

6. Émulsion d'huile dans eau gélifiée administrable par voie orale selon la revendication 4 ou la revendication 5, dans laquelle lesdits dérivés d'acide gras sont des glycérides, de préférence des triglycérides ; éventuellement dans laquelle lesdits dérivés d'acide gras sont des triglycérides à chaîne moyenne présentant deux ou trois acides gras à chaîne moyenne qui peuvent être identiques ou différents, de préférence des acides gras comprenant de 6 à 12 atomes de carbone, par exemple 8 ou 10 atomes de carbone.

7. Émulsion d'huile dans eau gélifiée administrable par voie orale selon l'une quelconque des revendications précédentes, dans laquelle la phase huileuse est dispersée dans la phase aqueuse gélifiée sous la forme de gouttelettes d'huile présentant un diamètre compris dans la plage de 10 nm à 100 µm, de préférence dans laquelle les gouttelettes d'huile présentent un diamètre moyen compris dans la plage de 100 nm à 1 µm.

8. Émulsion d'huile dans eau gélifiée administrable par voie orale selon l'une quelconque des revendications précédentes, dans laquelle ladite phase aqueuse comprend un agent gélifiant physiologiquement tolérable choisi parmi des alginates, des pectines, des carraghénanes, la gélatine, la gomme gellane et l'agar, de préférence dans laquelle l'agent gélifiant est la gélatine, éventuellement dans laquelle la gélatine est présente dans la phase aqueuse à une concentration comprise entre 5 % en poids et 25 % en poids sur la base du poids de la phase aqueuse.

9. Émulsion d'huile dans eau gélifiée administrable par voie orale selon l'une quelconque des revendications précédentes, dans laquelle la phase aqueuse comprend en outre un ou plusieurs agents gonflants, par exemple des alcools de sucre, de préférence dans laquelle lesdits agents gonflants sont présents à une concentration comprise entre 45 % en poids et 70 % en poids, de préférence entre 50 % en poids et 65 % en poids, par exemple entre 55 % en poids et 60 % en poids, sur la base de la phase aqueuse.

10. Émulsion d'huile dans eau gélifiée administrable par voie orale selon l'une quelconque des revendications précédentes qui comprend en outre un activateur d'assimilation oromucosal, de préférence dans laquelle ledit activateur d'assimilation oromucosal est un polymère cationique, plus de préférence du chitosane ou un dérivé de chitosane.

11. Boîtier comprenant un compartiment étanche à l'air et à la lumière contenant une unité de dose de l'émulsion d'huile dans eau gélifiée selon l'une quelconque des revendications précédentes.

12. Procédé de préparation d'une émulsion d'huile dans eau gélifiée administrable par voie orale sous forme de dose unitaire selon l'une quelconque des revendications 1 à 10, ledit procédé comprenant les étapes consistant à :
(i) former une phase huileuse qui comprend un ou plusieurs lipides physiologiquement tolérables et au moins un cannabinoïde ; former une phase aqueuse comprenant un agent gélifiant physiologiquement tolérable ; combiner ladite phase huileuse et ladite phase aqueuse pour former une émulsion d'huile dans eau ; et permettre à ladite émulsion de gélifier ; ou
(ii) former une phase huileuse qui comprend un ou plusieurs lipides physiologiquement tolérables ; former une phase aqueuse comprenant un agent gélifiant physiologiquement tolérable ; combiner ladite phase huileuse, ladite phase aqueuse et au moins un cannabinoïde pour former une émulsion d'huile dans eau ; et permettre à ladite émulsion de gélifier.

13. Émulsion d'huile dans eau gélifiée selon l'une quelconque des revendications 1 à 10 destinée à être utilisée par voie orale comme un médicament ou à être utilisée par voie orale en thérapie.

14. Émulsion d'huile dans eau gélifiée selon l'une quelconque des revendications 1 à 10 destinée à être utilisée par voie orale dans le traitement d'une condition en réponse à un cannabinoïde, dans laquelle ladite condition est choisie parmi une douleur (par exemple, douleur cancéreuse, douleur d'arthrite), une inflammation, des troubles neurologiques, des troubles de l'humeur (par exemple, anxiété), l'épilepsie, des troubles du sommeil, les symptômes de sclérose en plaques, l'anorexie, la schizophrénie, une maladie inflammatoire de l'intestin (par exemple, maladie de Crohn et colite ulcéreuse), et des nausées provoquées par chimiothérapie, de préférence dans laquelle ladite condition est choisie parmi une douleur chronique (par exemple, douleur musculo-squelettique) et des maladies inflammatoires chroniques (par exemple, arthrite rhumatoïde et ostéoarthrite).

15. Utilisation non thérapeutique d'une émulsion d'huile dans eau gélifiée selon l'une quelconque des revendications 1 à 10 en tant qu'un nutraceutique.
